# EUROPEAN PATENT APPLICATION

(11) **EP 0 997 537 A2**
(43) Date of publication of application: **03.05.2000**
(21) Application number: 99307236.2
(22) Date of filing: 13.09.1999
(51) Int. Cl.: C12Q 1/70, G01N 33/573

(54) **Assay for detecting modulators of serine/threonine phosphatease activity**

(30) Priority: 14.09.1998 GB 9820025; 27.07.1999 GB 9917631
(71) Applicant: Pfizer Limited, Sandwich Kent CT13 9NJ (GB); PFIZER INC., New York, N.Y. 10017 (US)
(72) Inventor: Ciaramella, Giuseppe, Sturry, Kent CT2 0JT (GB)
(74) Representative: Harding, Charles Thomas

(57) **Abstract**

An assay method for identifying an agent that can affect the activity or expression of a nucleotide sequence or the expression product (EP) thereof is described. The assay method comprises contacting an agent with a nucleotide sequence coding for a serine/threonine phosphatase; and/or the EP thereof (i.e. a serine/threonine phosphatase); and determining whether the agent affects the activity or expression of the nucleotide sequence and/or the EP.

## Description

The present invention relates to an assay.

In particular, the present invention relates to an assay for screening for putative agents that may be useful as anti-viral agents.

More in particular, the present invention relates to an assay for screening for putative anti-viral agents that may be useful for affecting protein kinase and/or phosphonase activity and, in doing so, cause an anti-viral effect.

Kinases are phosphotransferase enzymes that transfer a phosphate group, typically from ATP, from one entity to another. Protein kinases phosphorylate one or more hydroxyl or phenolic groups in proteins. By way of example, serine protein kinases phosphorylate serine groups in proteins.

According to the Oxford Dictionary of Biochemistry and Molecular Biology (Oxford University Press, 1997), two classes of protein kinases are recognised.

The first class of kinases are those that phosphorylate seryl or threonyl hydroxyls. This class contains enzymes that were classically identified as regulating pathways of intermediary metabolism (eg. glycogen phosphorylase kinase). Subsequently, protein kinase A (cyclic-AMP-dependent protein kinase) and protein kinase C were recognised in this class.

The second class of kinases are those that phosphorylate tyrosyl phenolic groups. The enzyme activity of this class is found in some cytokine receptors such as platelet-derived growth factor receptor and epidermal growth factor receptor and in onco-gene products such as that of SRC.

Over 110 unique gene products are known to make up the protein kinase superfamily. Important motifs of this superfamily include GXGXXG...AHK and APE...DXWSXG, which are common to both serine/theorine- and tyrosine-specific activities. The tyrosine-specific kinase activity associated with the cytoplasmic domains of certain cytokine receptors forms part of an important signal-transduction mechanism implicated in cell growth.

It is known that in virally infected cells, interferon (IFN) activates protein kinase (PKR) by phosphorylating it to PKR-P. PKR is sometimes known as P68 (sometimes written as p68). In addition, this IFN activatable PKR is sometimes referred to as being an interferon-induced dsRNA-activated kinase.

P68 has been found in cells infected by adenovirus, Epstein-Barr virus, influenza virus, picornavirus (such as poliovirus, mengovirus, encephalomyocarditis virus), human immunodeficiency virus (HIV), reovirus, and vaccinia virus.

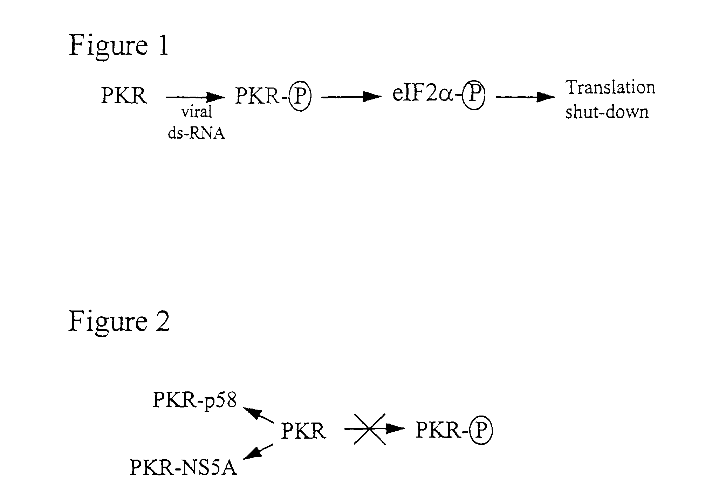

P68 activation takes place in virally infected cells. After P68 activation has occured in virally infected cells, the resultant PKR-P then phosphorylates (switches off) the translation initiation factor eIF2α, which leads to cell death (i.e. of the virus infected cell). This mechanism is schematically presented in Figure 1.

However, it is not uncommon for some viruses to modulate the PKR pathway - i.e. the pathway that leads to activation of P68 which in turn phosphorylates eIF2α. In this respect, some viruses can actually inhibit the activation of PKR to PKR-P. Hence, the virally infected host cells survive.

Up until now, viruses were known to modulate the PKR pathway by at least two mechanisms. Each of these mechanisms is presented schematically in Figure 2.

The first mechanism concerns use of a viral protein (e.g. NS5A). NS5A can form a complex with PKR. This resultant complex can be called NS5A/PKR.

Some teachings on NS5A may be found in WO-A-98/39487.

The second mechanism concerns the virus inducing the expression of the host cell protein P58 (sometimes referred to as p58). P58 can bind to PKR. The resultant complex can be called the P58/PKR complex.

Katze (*J. Interferon Research* (1992) 12 241-248) presents a review on interferon-induced dsRNA-activated kinase. The review starts by saying:
"To avoid decreases in protein synthesis rates during infection, animal viruses down-regulate activity of the interferon (IFN)-induced, double-stranded (ds) RNA-activated protein kinase. Furthermore, there seems to be a good, but not perfect, correlation between the ability of a virus to down regulate the kinase and its lack of susceptibility to IFN, suggesting this also may allow viruses to at least partially escape the antiviral effects of IFN. The dsRNA activated protein kinase will be referred to as P68 based on its *M*ᵣ of 68,000 in human cells, although it also has been referred to by others as dsI, DAI, dsRNA-PK, eIF-2-PK_{ds}, and P1/elF-2 kinase. The only other species in which the kinase has been well characterized is mice, where the kinase has a *M*ᵣ of 65,000; however, recently we identified kinase homologues in bovine and monkey cells as well (G.N. Barber and M.G. Katze, unpublished).
"The P68 protein kinase, recently molecularly cloned, is a serine/threonine kinase characterized by two distinct kinase activities: the first involves an autophosphorylation (activation) reaction and the second a protein kinase activity on exogenous substrates. Once activated by dsRNA, P68 phosphorylates its natural substrate, the α-subunit of protein synthesis eukaryotic initiation factor 2, eIF-2. Phosphorylation of the eIF-2 α-subunit blocks the eIF-2β-mediated exchange of GDP in the inactive eIF-2-GDP complex, with GTP required for catalytic utilization of eIF-2. These events lead to limitations in functional eIF-2, which is an essential component of protein synthesis and is normally required to bind initiator Met-tRNA (via the ternary complex eIF-2-GTP-Met-tRNA) to the initiating ribosomal subunit before mRNA is bound. Thus, activation of the kinase triggers a series of events that culminates in an inhibition of protein synthesis initiation. Since virus-specific RNAs synthesized during infection have the potential to activate P68, viruses must down-regulate kinase activity to replicate and survive."

Katze then says:
"Despite the many studies described above, we still know little about the molecular mechanisms of activation and repression of the P68 protein kinase by virus-specific RNAs. P68 does not possess the ribonucleoprotein consensus sequences typical of RNA-binding proteins, although the sequence indicates that the kinase is a hydrophilic protein that may account for its ability to bind dsRNA."

Some further studies on P68 are now reported on below.

Cai R, Williams BR (*J Biol Chem* 1998 May 1;273(18):11274-11280) report that the interferon-induced, double-stranded RNA (dsRNA)-activated protein kinase, PKR, inhibits protein synthesis *via* phosphorylation of the α subunit of the translation initiation factor eIF2. According to the authors, a kinase insert region N-terminal of PKR kinase subdomain V, which is conserved among eIF2α kinases, has been proposed to determine substrate specificity of these kinases. To investigate the function of this kinase insert region, selective PKR mutants were generated by the authors, and kinase activities and eIF2α affinities were analyzed *in vitro.* The *in vivo* function was investigated by growth inhibitory assays in yeast and translational assays in COS cells. Among the 13 mutations, 5 lost kinase activity and 3 exhibited less than 30% of wild-type eIF2α binding activity. The deletion of the conserved sequence (amino acids 362-370) resulted in a protein that had no kinase activity and only about 25% of wild-type eIF2α binding. The authors suggest that this sequence is not only required for PKR kinase activity but also is important for substrate interaction. The authors also determined that the hydrophobicity of the conserved sequence of PKR is required for kinase activity but is not crucial for eIF2α binding. The authors also showed that the amino acid residue Glu-367 in the conserved motif was shown to be directly involved in substrate binding but was not important for kinase activity. According to the authors these results suggest that the activation of PKR is not a prerequisite for its binding to the substrate and that the conserved motif in subdomain V contributes to the interaction of PKR and eIF2α.

Kimball SR, Horetsky RL, Jagus R, Jefferson LS *(Protein Expr Purif* 1998 Aspr; 12(3):415-419) report that the α-subunit of eukaryotic initiation factor eIF2 (eIF2α) plays an important role in the regulation of mRNA translation through modulation of the interaction of eIF2 and a second initiation factor, eIF2B. According to the authors, the interaction of the two proteins is regulated *in vivo* by phosphorylation of eIF2α at Ser51. In these studies, rat eIF2α was expressed in Sf21 cells using the baculovirus expression system. The recombinant protein was purified to >90% homogeneity in a single immunoaffinity chromatographic step. The protein was free of endogenous eIF2α kinase activity and was rapidly phosphorylated by the eIF2α kinases HCR and PFR. A variant of eIF2α in which the phosphorylation site was changed in Ala was also expressed and purified. The variant eIF2α was not phosphorylated by either HCR or PKR, demonstrating that the kinases specifically phosphorylate the correct site in the recombinant protein even in the absence of the other two subunits of the protein. The authors conclude that a rapid and inexpensive method for obtaining eIF2α has been developed - and that use of the wildtype and variant forms of eIF2α to measure eIF2α kinase activity in cell and tissue extracts should greatly facilitate examination of the regulation of mRNA translation under a variety of conditions.

Romano PR *et al (Mol Cell Biol* 1998 Apr; 18(4):2282-2297) report that the human double-stranded RNA-dependent protein kinase (PKR) is an important component of the interferon response to virus infection. According to the authors, the activation of PKR is accompanied by autophosphorylation at multiple sites, including one in the N-terminal regulatory region (Thr-258) that is required for full kinase activity. According to the authors, several protein kinases are activated by phosphorylation in the region between kinase subdomains VII and VIII, referred to as the activation loop. The authors show that Thr-446 and Thr-451 in the PKR activation loop are required *in vivo* and *in vitro* for high-level kinase activity. According to the authors, mutation of either residue to Ala impaired translational control by PKR in yeast cells and COS1 cells and led to tumour formation in mice. These mutations also impaired autophosphorylation and eukaryotic initiation factor 2 subunit α (eIF2α) phosphorylation by PKR *in vitro.* Whereas the Ala-446 substitution substantially reduced PKR function, the mutant kinase containing Ala-451 was completely inactive. PKR specifically phosphorylated Thr-446 and Thr-451 in synthetic peptides *in vitro,* and mass spectrometry analysis of PKR phosphopeptides confirmed that Thr-446 is an autophosphorylation site *in vivo.* Substitution of Glu-490 in subdomain X of PKR partially restored kinase activity when combined with the Ala-451 mutation. According to the authors, this finding suggests that the interaction between subdomain X and the activation loop, described previously for MAP kinase, is a regulatory feature conserved in PKR.

Sharp TV, Witzel JE, Jagus R *(Eur J Biochem* 1997 Nov 15; 250(1):85-91) report that the vaccinia virus K3L gene product, pK3, binds to the dsRNA-activated protein kinase, PKR, reducing its ability to interact with and phosphorylate eIF2α, several laboratories have utilized pK3 to investigate the molecular determinants that specify substrate recognition by PKR. According to the authors, the data demonstrate that the natural substrate, eIF2α, also binds to PKR *in vitro* and interacts with the same or an overlapping domain within PKR. A truncated form of eIF2α, representing the N-terminal 123 amino acids and containing the regions of homology to pK3, retains the ability to bind PKR.

Further discussion on eIF-2α may be found in US-A-5795713. This document discloses a method for identifying inducers and inhibitors of programmed cell death in a cell-free system that would be useful in preventing neurodegenerative conditions, Alzheimer's disease, amyotrophic lateral sclerosis, Huntington's disease, spinal muscle atrophy, and other neurodegenerative disorders. According to the authors, the method expolits the finding that programmed cell death is accompanied by shutdown of cellular protein synthesis and by phosphorylation of eIF-2α, and that the dephosphorylation of eIF-2α prevents the shutdown of protein synthesis.

Further teachings on P68 and other viral aspects may be found in US-A-5738985 - according to which: "the protein known as p68 protein kinase is an interferon-induced double-stranded RNA-activated protein kinase. This kinase is activated by the double-stranded RNA typically found in virus-infected cells. Once activated, the kinase phosphorylates the alpha subunit of the initiation factor eIF-2, an event which quickly leads to a block in the initiation stage of translation. The effect is to shut down protein synthesis in the cell, causing that cell to die: something which the multi-cellular infected organism can afford but which the virus cannot. To ensure continued translation in infected cells, different viruses have evolved a variety of mechanisms to prevent or counteract activation of the p68 kinase (reviewed in Katze, 1992). These include viral RNAs which bind to the kinase and prevent binding of the double-stranded RNA activator, as used by adenovirus (reviewed by Mathews M. B. & Shenk T., J. Virology 65, 5657-5662), HIV (Edery *et al.,* Cell 56, 303-312, 1989; Gunnery *et al.,* Proc. Natl. Acad. Sci. USA 87, 8687-8691, 1990; Roy *et al.,* J. Virology 65, 632-640, 1991) and Epstein-Barr virus (Clarke *et al.,* Eur. J. Biochem 193, 635-641, 1990; Clarke *et al.,* Nucl. Acids Res. 19, 243-248, 1991); viral proteins which bind the double-stranded RNA and prevent it binding to the kinase, as used by vaccinia virus and reovirus (Watson *et al.,* Virology 185, 206-216, 1991; Imani and Jacobs, Proc. Natl. Acad. Sci. USA 85, 7887-7891, 1988); viral proteins which act as pseudosubstrates of the kinase, as used by vaccinia virus (Beattie *et al.,* Virology 183,419-422, 1991); recruitment of a cellular protein, p58, to block activation of the kinase and inhibit active kinase, as used by influenza virus (Lee *et al.,* 1990); and recruitment of a cellular protein into a complex with RNA (possibly viral double-stranded RNA) which degrades p68, as used by poliovirus (Black *et al.,* J. Virology, 67, 791-800, 1993). P68, and the RNAs and proteins just described which interact with it, are examples of a broader class of macromolecules which have been shown to be involved in the seizure or retention by viruses of control of translation in infected cells. Other examples include: the host translational factors eIF2 and eIF3/4B, which are reported to be impaired in cells infected with vesicular stomatitis virus (VSV) (Centrella and Lucas-Lenard, J. Virology 41, 781-791, 1982; Thomas and Wagner, Biochemistry 22, 1540-1546, 1983); the product of the VSV gene "P", reportedly responsible for host translational inhibition (Stanners, *et al.,* Cell 11, 273-281, 1977); the poliovirus 2A protease, responsible for degrading the p220 subunit of cap-binding protein complex (eIF-4F) in infected cells, and thereby preventing cap-dependent translation of host-cell mRNAs (Etchison *et al.,* J. Biol. Chem. 257, 14806-14810, 1982); the cellular protease recruited/activated by the poliovirus protease 2A to cleave p220 (the poliovirus enzyme does not cleave p220 directly) (Lloyd *et al.,* Virology 150,299-303, 1986); the p220 protein degraded in poliovirus-infected cells; the host initiation factor eIF-4E, another component of the cap-binding protein complex which is dephosphorylated in adenovirus-infected cells to shut off host protein synthesis (Huang and Schneider, Cell 65, 271-280, 1991); and the cellular proteins p57 (also known as polypyrimidine tract-binding protein, pPTB), p50 and p52 implicated in the initiation of translation at internal ribosome entry sites within poliovirus and other viral RNAs (Jang and Wimmer, 1990; del Angel et. al., Proc. Natl. Acad. Sci. USA 86, 8299-8303, 1989; Meerovitch *et al.,* Genes Dev. 3, 1026-1034, 1989; Najita and Sarnow, Proc. Natl. Acad. Sci. USA 87, 5846-5850, 1990). To these examples can be added a variety of macromolecules used by viruses to cut off the supply of host-cell mRNAs and/or favor the production of viral RNAs in infected cells. These include: the vhs gene product of herpes simplex virus (HSV), a virion protein which degrades mRNAs in infected cells (Kwong and Frenkel, Proc. Natl. Acad. Sci. USA 84, 1926-1930, 1987; Kwong *et al.,* J. Virology 62, 912-921, 1988); another HSV virion protein which binds to a sequence-specific DNA-binding protein in host cells, causing increased transcription from viral gene promoters (Campbell *et al.,* J. Mol. Biol. 180, 1-19, 1984); a cap-dependent endonuclease encoded by influenza virus which cleaves nascent host-cell transcripts in the nucleus to provide primers for the synthesis of viral mRNA from the viral RNA genome (Bouloy *et al.,* Proc. Natl. Acad. Sci. USA 75, 4886-4890, 1978; Plotch *et al.,* Cell 23, 847-858, 1981); nucleases used by influenza virus and poxvirus to degrade host-cell mRNAs (Rice and Roberts, J. Virology 47, 529-539, 1983; Inglis SC, Mol. Cell. Biol 2, 1644-1648, 1982); viral inhibitors of host-cell RNA polymerase II (the enzyme responsible for transcription of host-cell mRNAs) such as the "positive-strand leader RNA" believed to bind a host-cell factor and prevent its binding to host-cell promoters (Grinell and Wagner, J. Virology 48, 88-101, 1983); and adenovirus proteins E1B-55K and E4-34K which inhibit transport of host-cell transcripts from the nucleus to the cytoplasm (Babiss *et al.,* Mol. Cell. Biol. 5, 2552-2558, 1985; Halbert *et al.,* J. Virology 56, 250-257, 1985; Pilder *et al.,* Mol. Cell. Biol. 6, 470-476, 1986). The above are all examples of a broader class of macromolecules involved in translation whose concentrations and/or activities are subject to modulation by viruses.... A variety of procedures are available to those skilled in the art which enable them to identify other such macromolecules (including polypeptides, proteins, glycoproteins, lipids, carbohydrates, mucopolysaccharides, glycolipids, and nucleic acids), and to design methods for selecting compounds which can prevent or moderate the interaction between viruses and these macromolecules. In general, the steps required include: ascertaining whether translation is modulated by a given virus during infection; identifying the specific macromolecule(s) mediating the effect on translation; identifying any other cellular and/or viral components involved; characterizing the interaction between these components; and designing a screening method in which disruption or moderation of this interaction can be detected. These steps can be performed in any sequence depending on the nature of the results obtained, and not all steps may be required in order to select compounds which can have the desired effect. ... Many of the procedures used are collected in such reference texts as Ausubel *et al.,* (eds) Current Protocols in Molecular Biology, Wiley-Interscience, New York, 1991, and Sambrook *et al.,* Molecular Cloning: A Laboratory Manual (2nd Ed.), Cold Spring Harbor Laboratory Press, Cold Spring Harbor, N.Y., 1989."

It is well known that viruses replicate inside cells by making use of cellular mechanisms of the host cells for replication. In this respect, there appear to be a number of events that could be targetted by an antiviral agent. These viral specific events include: attachment of the virus to the cell membrane; penetration of the virus into the cells; uncoating of the virus; viral nucleic acid synthesis; viral protein synthesis and maturation; and assembly and release of infectious particles (Drug and Market Development, Vol 3. No. 9, pp. 174-180 (Feb. 15, 1993)).

A feature of viral protein synthesis is that use is made of the host cell ribosomes and other host components. An example of such a host component is a protein called P58 (which is discussed further herein). Thus, protein synthesis inhibitors may be equally likely to be harmful to the host cell as they are to the virus itself. So, in the development of an antiviral therapeutic there is often a balance between not harming the host and destroying, or at least arresting the replication of, the virus (Drug and Market Development, Vol 3. No. 9, pp. 174-180 (Feb. 15, 1993)).

A number of agents are now used to overcome or suppress viral infection. These agents include naturally-occurring proteins - such as interferon - and other chemical compounds - such as AZT. These agents do not have, however, a universal applicability - as it is known that interferons have some efficacy with respect to some viral diseases and AZT is used in respect of an immunodeficiency disease caused by the virus HIV-1. Thus, there is still a need for screening for new anti-viral agents.

"Screening" typically means a process in which a number of potentially useful agents are processed to see if they possess favourable properties.

Some methods for screening for agents useful for the treatment of infection are known. By way of example, further reference may be made to US-A-5738985 which discloses a method for screening for an antiviral agent. That method comprises determining whether a potential agent interacts with a virus or cellular component which allows or prevents preferential translation of a virus RNA compared to a host RNA under virus infection conditions; and determining whether any interaction of the agent with the component reduces the level of translation of an RNA of the virus. A preferred assay of US-A-5738985 concerns determining whether a potential antiviral agent is effective to inhibit viral replication in a host eukaryotic cell, where the virus produces an inhibitor which interferes with the activation or activity of the host-cell interferon-induced, double-stranded RNA-activated protein kinase, comprising incubating a mixture containing the protein kinase, the viral inhibitor, and the agent to be tested under conditions effective to cause inhibitor interference with the activation or activity of the protein kinase, and examining the mixture for such interference.

As indicated above, interferons (IFN) are known to have a broad antiviral effect. This particular phenomenon is reviewed in Vilcek and Sen (1996). However, unfortunately their effectiveness as therapeutic drugs is often compromised by the fact that many viruses have evolved ways to interfere with the molecular interactions necessary to elicit an IFN antiviral response (again, see Table 1).

**Table 1**

| **Virus** | **Inhibitor** |
|---|---|
| Vaccinia | K3L |
| HSV-1 | γ₁ 34.5 |
| Influenza | NS1 |
| Reo and Rotaviruses | σ3 protein; s1 RNA |
| Epstein-Barr Virus | EBERS1 & 2; W1W2 exons |
| Adenovirus | VA1, Activated p58 |
| Poliovirus | PKR protease |
| HIV-1 | Tar |
| HCV | NS5A |

The antiviral effect of IFN on viruses such as picornaviruses, reoviruses, adenovirus, vaccinia, vescicular stomatitis virus, influenza, and potentially HCV, is achieved *via* inhibition of translation, with consequent death of the infected cell. At least two IFN-induced enzyme pathways lead to inhibition of protein synthesis: the ds-RNA dependent protein kinase (PKR) pathway and the 2'-5' oligoadenylate synthase/RNAse L pathway.

With respect to the PKR pathway, and as mentioned above, activation thereof leads to the phosphorylation of the translation initiation factor eIF2α (again, see Figure 1). This phosphorylated form of eIF2α is inactive in the sense that it is unable to initiate cellular translation. Consequently, the cell rapidly dies. The enzyme that catalyzes the phosphorylation of eIF2α is the phosphorylated form of PKR, whose expression is induced by IFN (reviewed in Clemens and Elia 1997). The specific antiviral effect of IFN is ensured by the fact that activation (i.e. phosphorylation) of PKR is dependent upon the presence of ds-RNA, a viral by-product. Inhibition of translation, and the subsequent cell death, therefore, can only occur in those cells that have been infected by the virus.

A potential HCV-specific target related to the PKR pathway is the interaction between the HCV NS5A protein and PKR. A relatively small (ca. 35 amino acid) sequence of this protein has been shown to correlate with response to IFN. As a protein-protein interaction, this target poses greater obstacles to the development of small molecule inhibitors.

The 2'-5' oligoadenylate synthetase (2-5(A)synthase)/RNAse L pathway causes degradation of cellular and viral mRNA through the action of the IFN-induced enzyme and RNAse L. Like for the PKR pathway, the active form of 2-5(A) synthase requires ds-RNA as its only cofactor. This pathway has been shown to be responsible for inhibiting the replication of picornaviruses, to which HCV is related, in IFN-treated cells. To the picornaviridae family of viruses belong several human pathogens that cause a variety of conditions that range from minor malaise to polyomelitis. A particular group of viruses that belong to this family, are known as coxsackieviruses. The group B of these viruses are being increasingly recognized as a cause of primary myocardial disease in adults and children.

The most cogent evidence establishing the anti-picornaviral function of this pathway has come from molecular genetic studies. Constitutive expression of a transfected 2-5(A) synthase cDNA clone was shown to be sufficient, without IFN treatment, for inhibiting picornaviruses (Chebath, Benech *et al.* 1987). In this pathway, the IFN-induced 2-5(A) synthase, catalyzes the synthesis of 2'-5'-linked oligoadenylates (2-5(A)) which activate a latent ribonuclease, RNAse L, present in all animal cells. Like in the PKR pathway, the cellular pool of the secondary messenger molecule, in this case 2-5(A), is regulated in most cells by another enzyme that catalyzes its hydrolysis, the 2-5(A) phosphodiesterase.

Sometimes a single approach may be effective against a viral pathogen. However, and because of the possibility of developed resistance to previously effective antiviral compounds, there remains a need for new antiviral agents with novel mechanisms of action and improved or different activity profiles.

There is also a need for agents which are active against viruses but are not toxic to mammalian cells, as toxicity to mammalian cells can lead to a low therapeutic index and undesirable side effects in the host (patient).

Despite the increased use of rational drug design, a preferred method continues to be the mass screening of compound "libraries" for active agents by exposing cultures of viral pathogens to the test compounds and assaying for inhibition of growth. In testing thousands or tens of thousands of compounds, however, a correspondingly large number of viral cultures must be grown over time periods which are relatively long compared to most bacterial culture times. Moreover, a compound which is found to inhibit viral growth in culture may be acting not on the desired target but on a different, less unique viral component, with the result that the compound may act against host cells as well and thereby produce unacceptable side effects.

Consequently, there is a need for assay or screening methods which more specifically identify those agents that are active against a certain intracellular target.

Additionally, there is a need for assay methods having greater throughput, *i.e.,* which reduce the time and materials needed to test each compounds of interest.

The present invention seeks to provide targets for identifying potential agents for the effective treatment of opportunistic infections caused by one or more viral species, which may be related.

The present invention seeks to provide a nucleotide sequence and/or its expression product (EP) thereof as targets for antiviral therapy as well as targets in assays for the identification of one or more agents which can act as antiviral agents.

The present invention also seeks to provide screening methods for identifying anti-viral agents.

The present invention also seeks to provide an assay that can enable workers to screen for *inter alia* anti-viral agents, in particular small molecule inhibitors.

Aspects of the present invention - including preferred embodiments thereof - are presented in the accompanying claims.

Thus, in one aspect the present invention provides a screen that is useful for identifying potential anti-viral agents. The screen makes use of a serine/threonine phosphatase.

The term "serine/threonine phosphatase" as used herein with reference to the present invention includes a complete serine/threonine phosphatase enzyme *per se,* or just active fragments or units thereof, such as sub-units thereof, or combinations thereof.

By way of example, active fragments/units of the serine/threonine phosphatase include the catalytic subunit and/or regulatory subunit. These active fragments/units may be sub-units. The active fragments may be the subunits *per se* (i.e. on their own) or they can located in or associated with other amino acid sequences (such as being contained within the complete sequence of a serine/threonine phosphatase) or they may be free of other serine/threonine phosphatase enzyme sequences (i.e. on their own or in proteins that do not contain all of the sequence for the serine/threonine phosphatase enzyme).

Thus, in accordance with the present invention it is possible to utilise active fragments/subunits of a serine/threonine phosphatase - such as the catalytic subunit thereof and/or the regulatory subunit thereof (sometimes referred to as the targeting sub-unit) - instead of the complete serine/threonine phosphatase enzyme. Likewise, it is also possible to utilise nucleotide sequences coding for those active fragments/subunits - i.e. nucleotide sequences that encode the catalytic subunit and/or regulatory subunit - instead of the nucleotide sequence coding for the complete serine/threonine phosphatase enzyme.

Thus, in accordance with the present invention, the reference to the serine/threonine phosphatase of the present invention includes a reference to the enzyme itself. In some cases, the reference to the serine/threonine phosphatase of the present invention just means a reference to the enzyme itself.

For some applications, preferably, however, the reference to the serine/threonine phosphatase of the present invention means sequences which encompass the catalytic subunit thereof and/or the regulatory subunit thereof.

For some applications, preferably, the reference to the serine/threonine phosphatase of the present invention means sequences which just encompass the catalytic subunit thereof and the regulatory subunit thereof.

In a preferred aspect, the reference to the serine/threonine phosphatase of the present invention encompasses at least the catalytic subunit thereof.

In a preferred aspect, the reference to the serine/threonine phosphatase of the present invention encompasses at least the catalytic subunit PP1α.

In one preferred aspect, the reference to the serine/threonine phosphatase of the present invention encompasses just the catalytic subunit PP1α.

The catalytic sub-unit may be used on its own or in the presence of other catalytic subunit(s) and/or one or more regulatory units and/or targetting units.

In a preferred aspect, the reference to the serine/threonine phosphatase of the present invention encompasses at least a regulatory subunit (or targetting sub-unit) which is normally associated with PP1α.

In one preferred aspect, the reference to the serine/threonine phosphatase of the present invention encompasses just the regulatory subunit (or targetting sub-unit) which is normally associated with PP1α.

The regulatory sub-unit may be used on its own or in the presence of other regulatory sub-unit(s) and/or one or more catalytic sub-unit regulatory units.

Thus, in a highly preferred aspect, the screen makes use of PP1α and/or a tagetting sub-unit normally associated with PP1α. In this respect, PP1α can be used on its own, the tagetting sub-unit can be used on its own, or the PP1α can be used in combination with - such as when bound to - the tagetting sub-unit with which it is normally associated. If the target is eIF2α then the targetting sub-unit can be GADD34. Teachings on GADD34 may be found in Hollander *et al* 1997 J Biol Chem 272 pp 13731-13737 and US-A-5795713. The targetting sub-unit may be the targetting sub-unuit for PKR.

Even though both US-A-5795713 and He *et al* (1997 PNAS Vol 94 pp 843-848) mention PP1α, it is to be noted that those teachings (i.e. those of US-A-5795713 and He *et al*) only refer to providing a screen to identify substances that would be useful in preventing neurodegenerative conditions, Alzheimer's disease, amyotrophic lateral sclerosis, Huntington's disease, spinal muscle atrophy, and other neurodegenerative disorders. Thus, it is to be noted that the teachings of both US-A-5795713 and He *et al* (*ibid*) neither disclose nor suggest a method for screening for putative anti-viral agents. Also, the teachings of both US-A-5795713 and He *et al (ibid)* do not disclose a method for screening for putative anti-viral agents using a cell based assay. Hence, in contrast to the teachings of both US-A-5795713 and He *et al (ibid)* we have found that it is possible to produce an anti-viral effect by modulating serine/threonine phosphatase activity, preferably PP1α activity.

We believe that to date there has been considerable evidence suggesting the importance of PKR in the cellular antiviral response. In fact, the list of animal viruses that have evolved various strategies to repress PKR activity is extensive and continues to grow - such that there may be a good correlation between the ability of a virus to inhibit PKR and resistance to the antiviral effects of IFN. We believe that this mechanism contributes to the poor response to IFN treatment (~25%) observed in patients infected with HCV, confirming the importance of PKR in the IFN pathway. In addition, PKR knockout mice exhibit a diminished antiviral response to encephalomyocarditis virus (EMCV) infection. Also, a similar diminished response to EMCV infection is observed in mouse embryo fibrolasts devoid of PKR.

According to one aspect of the present invention there is provided an assay method for identifying an agent that can affect the activity or expression of a nucleotide sequence or the expression product (EP) thereof, the assay method comprising contacting an agent with: a nucleotide sequence coding for a serine/threonine phosphatase; and/or the EP thereof (i.e. a serine/threonine phosphatase); and determining whether the agent affects the activity or expression of the nucleotide sequence and/or the EP.

According to another aspect of the present invention there is provided an assay method for identifying an agent that can affect serine/threonine phosphatase activity or expression thereof, the assay method comprising contacting an agent with an expression product (EP) which is a serine/threonine phosphatase; and determining whether the agent binds to or otherwise interacts with and activates or inhibits an activity of the EP; wherein the presence or absence of a signal generated from the binding or interaction of the agent with the EP enzyme or nucleotide sequence encoding same is indicative that the agent can affect the EP activity or expression.

According to another aspect of the present invention there is provided a process comprising the steps of: (a) performing the assay according to the present invention; (b) identifying one or more agents that do affect the EP activity or expression; and (c) preparing a quantity of those one or more identified agents.

In accordance with the present invention there is also provided a modulator of an interaction between a serine/threonine phosphatase and PKR-P and/or eIF2α-P, wherein the modulator is capable of exhibiting anti-viral effects.

The term "treatment" as used herein with reference to the present invention includes a therapeutic effect and/or a prophylatic effect. Typically, the anti-viral effect will be a prophylatic effect and/or a therapeutic effect. In a preferred embodiment, the assay method of the present invention is used to screen for agents useful in the therapeutic treatment of viral infections.

The treatment may be for any animal. However, in a preferred embodiment, the treatment will be for a human.

The agent may affect the activity or expression of the nucleotide sequence coding for the serine/threonine phosphatase by binding to the sequence itself - either the sense strand or the anti-sense strand - or by interacting with the corresponding RNA sequence. Alternatively, or in addition, the agent may modulate the expression of the nucleotide sequence by interacting with, for example, one or more of the expression control elements - such as the promoter associated with the sequence coding for the serine/threonine phosphatase. Alternatively, or in addition, the agent may modulate the expression of the nucleotide sequence by interacting with any signalling molecule that leads to upregulation of the expression of the serine/threonine phosphatase.

In accordance with the present invention we have found that a putative anti-viral agent may affect the activity or expression of the serine/threonine phosphatase by binding to it and thereby preventing the serine/threonine phosphatase from affecting the activation or activity of P68.

Preferably, the agent may affect the activity or expression of the serine/threonine phosphatase by binding to it. and thereby preventing the serine/threonine phosphatase from affecting the activity of the activated P68.

In addition, or in the alternative, the agent may affect the activity or expression of the serine/threonine phosphatase by binding to it and thereby preventing the serine/threonine phosphatase from affecting the activation or activity of eIF2.

In addition, or in the alternative, preferably the agent may affect the activity or expression of the serine/threonine phosphatase by binding to it and thereby preventing the serine/threonine phosphatase from affecting the activity of the activated eIF2.

In a further aspect, the invention provides a method of treating a subject comprising administering to the subject a therapeutically effective amount of an agent identified by the assay method of the present invention or an agent corresponding thereto.

The phrase "agent corresponding thereto" as used herein means that the agent that is used as the therapeutic need not be the actual agent that was used in the assay screen - but is an agent having the same chemical structure as that agent which was used in the screen.

The present invention also relates to novel antiviral agents identified by the assay method according to the present invention. It also includes novel pharmaceutical compositions which include antiviral agents identified by the assay method of the present invention.

The present invention also covers constructs comprising a sequence coding for the phosphatase of the present invention or an active portion thereof linked (directly or indirectly) to a nucleotide sequence coding for a reporter polypeptide.

The construct may be contained within a cell, preferably a eukaryotic cell.

The construct may be, or may be part of, an expression vector or expression system.

In this respect, a number of expression systems can be used to produce or express the sequences of the present invention. Such vectors include chromosomal, episomal and virus-derived vectors. For example, the vectors may be derived from bacterial plasmids. In the alternative or in addition, the vectors may be derived from yeast chromosomal elements. In the alternative or in addition, the vectors may be derived from bacteriophage. In the alternative or in addition, the vectors may be derived from viruses such as baculoviruses, papova viruses, such as vaccinia viruses, SV40, pseudorabies viruses adenoviruses, fowl pox viruses, and retroviruses. In the alternative or in addition, the vectors may be derived from transposons. In the alternative or in addition, the vectors may be derived from yeast episomes. In the alternative or in addition, the vectors may be derived from insertion elements. In the alternative or in addition, the vectors may be derived from vectors derived from combinations thereof, such as those derived from plasmid and bacteriophage genetic elements.

The constructs of the present invention may contain control regions that regulate expression. Generally, any system or vector suitable to maintain, propagate or express polynucleotides and/or to express a polypeptide in a host may be used for expression in this regard. The appropriate DNA sequence may be inserted into the expression system by any of a variety of well-known and routine techniques.

The present invention also provides a method of inhibiting viral replication in a host eukaryotic cell comprising administering an agent identified by the assay method of the present invention or an agent corresponding thereto.

In a related aspect, the invention also provides a modified virus which is capable of producing an agent that is capable of modulating a host component that is able to affect P68 activity. In addition, or in the alternative, the invention also provides a modified virus which is capable of producing an agent that is capable of modulating a host component that is able to affect eIF2 activity.

The present invention also provides a method for screening for agents that are capable of inhibiting viral replication in a host eukaryotic cell, wherein the virus is capable of upregulating a serine/threonine phosphatase that interferes with the activity of the host-cell interferon-induced, double-stranded RNA-activated protein kinase. The method includes incubating a mixture containing the protein kinase, the serine/threonine phosphatase, and the agent to be tested, under conditions effective to cause inhibitor interference with the activity of the protein kinase, and determining whether such interference occurs.

In one preferred embodiment, the protein kinase and the serine/threonine phosphatase according to the present invention are expressed in a yeast cell. In this aspect, the invention features a yeast cell for use in screening for putative anti-viral agents effective to inhibit viral replication in a host eukaryotic cell, where the virus upregulates expression of serine/threonine phosphatase that interferes with the activity of the host-cell interferon-induced, double-stranded RNA-activated protein kinase. The cell may include (1) an expressable sequence(s) coding for a mammalian interferon-induced, double-stranded RNA-activated protein kinase and/or eIF2; (b) an expressable reporter sequence gene whose expression in increased by activation of the protein kinase; and (c) an expressable sequence coding for a serine/threonine phosphatase that is able to affect the activation of the protein kinase and/or eIF2.

One key aspect of the present invention is that it enables workers to identify or select agents that could overcome or suppress viral suppression of interferon-induced cell death.

In one preferred aspect of the present invention, the assay method comprises forming an assay mixture comprising the agent or agents to be tested; a nucleotide sequence coding for the serine/threonine phosphatase and/or the serine/threonine phosphatase itself; and eIF2α (which may be in an activatable form or may be in activated form). In this assay method, one still determines whether the agent affects the activity or expression of the nucleotide sequence and/or the serine/threonine phosphatase. However, this can be achieved by determining the activity of the serine/threonine phosphatase itself and/or determining the activity of the eIF2α (which may or may not be a phosphorylated form).

In an other preferred aspect of the present invention, the assay method comprises forming an assay mixture comprising the agent or agents to be tested; a nucleotide sequence coding for the serine/threonine phosphatase and/or the serine/threonine phosphatase itself; and P68 (which may be in an activatable form or may be in activated form) and/or eIF2 (which may be in an activatable form or may be in activated form). In this assay method, one still determines whether the agent affects the activity or expression of the nucleotide sequence and/or the serine/threonine phosphatase. However, this can be achieved by determining the activity of the serine/threonine phosphatase itself and/or determining the activity of the P68 (which may or may not be in a phosphorylated form) and/or eIF2 (which may be in an activatable form or may be in a phosphorylated form).

In one embodiment, the assay method comprises forming an assay mixture comprising the agent or agents to be tested; a nucleotide sequence coding for the serine/threonine phosphatase and/or the serine/threonine phosphatase itself; P68 (which may be in an activatable form or may be in activated form); and eIF2α (which may be in an activatable form or may be in activated form). In this assay method, one still determines whether the agent affects the activity or expression of the nucleotide sequence and/or the serine/threonine phosphatase. However, this can be achieved by determining the activity of the serine/threonine phosphatase itself and/or determining the activity of the P68 (which may or may not be a phosphorylated form) and/or determining the activity of the eIF2α (which may or may not be a phosphorylated form).

If the assay utilises P68, then that protein kinase can be prepared from a number of sources. By way of example, the protein kinase can be prepared from interferon-induced mammalian cells, preferably from interferon-induced human cells. Alternatively, or in addition, the P68 can be prepared by recominant DNA techniques. In this respect, if the assay occurs in a host cell then that host cell may prepare heterologous P68.

As indicated above, there are a number of literature references on how to prepare P68.

By way of example, US-A-5738985 states that: "p68 protein kinase can be prepared from interferon-induced human tissue culture cell lines. In this respect, cells can be lysed by Dounce homogenization, and nuclei and cell debris removed by centrifugation at 30,000xg for 20 minutes. 4M KCl is added to the supernatant to a final concentration of 100 mM, and ribosomes are pelleted by centrifugation at 60,000 rpm in Beckman type 60 rotor. The ribosomal pellet is resuspended in 800 mM KCl, 20 mM HEPES (pH 7.4), 1.5 mM MgCl₂, 0.1 mM EDTA, 1 mM DTT and 1 µM phenylmethylsulfonyl fluoride (PMSF), then homogenized using a Dounce homogenizer. The ribosomes are then centrifuged again at 60,000 rpm for 90 min at 4°C in a type 60 rotor. The resulting supernatant is dialyzed against 50 mM KCl, 20 mM HEPES (pH 7.4), 1.5 mM MgCl₂, 0.1 mM EDTA, 1 mM DTT, 10% glycerol and 1 µM PMSF. The dialysate is centrifuged again to remove solids. The resulting supernatant (ribosomal salt wash) is applied to a DEAE-cellulose column equilibrated in 50 mM KCI, 20 mM HEPES (pH 7.4), 1.5 mM MgCl₂, 0.1 mM EDTA, 1 mM DTT, 10% glycerol and 1 µM PMSF. p68 kinase is collected in the flow through fraction, adjusted to pH 6.8, and applied to a S-Sepharose Fast Flow (Pharmacia) column equilibrated with 50 mM KCl, 20 mM HEPES (pH 6.8), 1.5 mM MgCl₂, 0.1 mM EDTA, 1 mM DTT, 10% glycerol and 1 µM PMSF. p68 kinase is eluted from the column in a linear gradient of 50-500 mM KCl in 20 mM HEPES (pH 7.4), 1.5 mM MgCl₂, 0.1 mM EDTA, 1 mM DTT, 10% glycerol and 1 µ.M PMSF. The p68 kinase peak is loaded onto a hydroxyapatite HPHT (BioRad) column equilibrated in 50 mM KCl, 20 mM HEPES (pH 7.2), 50 mM potassium phosphate (pH 7.2), 1.5 mM MgCl₂, 0.1 mM EDTA, 1 mM DTT, 10% glycerol and 1 µM PMSF. p68 kinase is eluted in a linear gradient of 50-500 mM potassium phosphate (pH 7.2). The p68 peak is loaded to an HR 5/10 Mono S column (Pharmacia) and eluted in a linear gradient of 50-500 mM KCl in 20 mM HEPES (pH 7.4), 1.5 mM MgCl₂, 0.1 mM EDTA, 1 mM DTT, 10% glycerol and 1 µM PMSF. The purified p68 is stored at -70°C."

US-A-5738985 also provides information on how to prepare P68 from recombinant *E. coli* cells.

In this respect, US-A-5738985 states that: "p68 is purified from *E.coli* expressing human p68 kinase, according to published methods (Barber *et al.,* 1991, Biochemistry 30:10356). Briefly, *E. coli* strain BL21 (DE3) pLysS is transformed with a plasmid containing the coding sequence for wild-type p68 protein kinase under the control of an inducible promoter. The resulting *E. coli* strain is grown to log phase, then induced to express p68 kinase. Cells are harvested by centrifugation, and lysed by lysozyme. p68 kinase is purified from the lysate by affinity chromatography using a monoclonal antibody to p68 kinase coupled to Sepharose, according to published methods (Galabru *et al.,* 1989, Eur. J. Biochem. 178:581)".

If the assay utilises eIF2α, then that protein can be prepared from a number of sources. By way of example, the protein can be prepared from interferon-induced mammalian cells, preferably from interferon-induced human cells. Alternatively, or in addition, the eIF2α can be prepared by recominant DNA techniques. In this respect, if the assay occurs in a host cell then that host cell may prepare heteologous eIF2α. There are a number of literature references on how to prepare eIF2α. For example, teachings on the eIF-2a protein kinases, which are regulators of translation in eukaryotes from yeasts to humans, may be found in J. Bio. Chem. 268: 7603-7606. A further reference is Scheper *et al.,* 1991, Biochem. Biophys. Acta 1089, 220-226.

Utilising the assay of the present invention it is possible to identify agents that may augment an IFN antiviral response by modulating the cellular pools of proteins involved in the pathway. Considering the generic effect of IFN, this approach could potentially lead to the development of broad-spectrum antiviral compounds. In addition, and together with agents that inhibit the NS5A/PKR interaction, it may also be able to develop alternative anti-HCV therapies.

More in particular, in accordance with the present invention, there is provided an assay that enables workers to identify or select agents that could be used to affect some molecular aspects of the IFN pathway, especially where small molecule inhibitors can be expected to augment the effectiveness of IFN as antiviral therapeutics. In particular, the assay of the present invention enables workers to identify or select agents against the target protein phosphatase, especially those that comprise as their catalytic domain PP1α, including the coding sequences therefor or the expression control elements therefor.

With the preferred aspect of the present invention relating to PP1, in particular PP1α and/or the targeting sub-unit normally associated with PP1α, we believe that an infected host cell modulates the activity of the activated PKR-P, by use of the cellular protein PP1α which dephosphorylates the PKR-P to PKR. Thus, for example, if the cell were to be substantially loaded with virus that to induce cell death may cause unnecessary further spread of the virus, the cell may deactivate the PKR-P by PP1α. By screening for agents that can modulate this PP1/PKR-P interaction should reveal putative anti-viral agents.

Thus, the present invention relates to methods for screening for agents which are useful in combatting or suppressing viral infection. In the general sense, the assay screening method of the present invention includes the step of bringing putative anti-viral agents into contact with serine/threonine phosphatase or the nucleotide sequence coding for same or the expression control elements therefor in order to ascertain whether any one or more of those agents can be used to combat or suppress viral infection.

The assay screening method of the present invention may also be used to investigate required or desirable dosage levels of known or identified anti-viral agents wherein the agents are brought into contact with serine/threonine phosphatase or the nucleotide sequence coding for same or the expression control elements therefor in order to ascertain which dose levels are preferable for combatting or suppressing viral infection.

The present invention also relates to the use of serine/threonine phosphatase or the nucleotide sequence coding for the same or the expression control elements therefor in the diagnosis and/or treatment of disease.

The present invention also relates to the use serine/threonine phosphatase or the nucleotide sequence coding for same or the expression control elements therefor as targets to evaluate and/or to screen for agents that can modulate viral activity.

The present invention further relates to genetically engineered host cells that comprise or express serine/threonine phosphatase or the nucleotide sequence coding for same or the expression control elements therefor that are useful to evaluate and/or to screen for agents that can modulate viral activity.

The assay method of the present invention may include use of the serine/threonine phosphatase or the nucleotide sequence coding for same or the expression control elements therefor linked to a reporter group (such as a polypeptide).

In one aspect the assay method of the present invention includes contacting the target sequence(s) with a potential antiviral agent under conditions which allow, for example, preparation of the reporter group in the absence of the agent. Then one determines whether the agent reduces the level of preparation of the reporter group. Hence, any agent which reduces this level is potentially a useful antiviral agent.

In another aspect the assay method of the present invention includes contacting the target sequence(s) with a potential antiviral agent under conditions which only allow, for example, preparation of the reporter group in the presence of the agent. Then one determines whether the agent increases the level of preparation of the reporter group. Hence, any agent which increases this level is potentially a useful antiviral agent.

In accordance with the present invention, preferably the serine/threonine phosphatase is PP1.

More preferably, the serine/threonine phosphatase is the catalytic domain PP1α and/or the targeting sub-unit normally associated with PP1α.

PP1 and PP1α are known entities. Typically, the native structure of PP1 is a 1:1 complex between the catalytic and a number of regulatory subunits. The role of these regulatory subunits is to target the catalytic subunit towards specific subcellular and to increase activity towards particular substrates.

PP1, and in particular PP1α, has been described by Takizawa N *et al (J Biochem (Tokyo)* 1997 Oct; 122(4):730-737). Here, the authors report that in response to stimulation of B-cells through cell surface IgM, the activity of the serine/threonine protein phosphatase PP1, but not PP2A, was transiently decreased and reached a minimum 10-20 min after the stimulation. The decrease was more profound in the immature B-cell line WEHI-231, than in the mature B-cell line BAL-17. Under these conditions, PP1α, an isoform of PP1, showed unique alterations in the patterns of several spots with distinct isoelectic points in the Western blot after two-dimensional eletrophoresis, whereas another isoform, PP1δ, did not shown any alteration.

PP1, in particular PP1α, has even been identified from a number of sources. For example, Solow B, Young JC, Kennelly PJ *(J Bacteriol* 1997 Aug; 179(16):5072-5075) report that with oligonucleotides modelled after conserved regions within the protein-serine/threonine phosphatases (Pps) of the PP1/2A/2B superfamily, the gene for the archaeal protein phosphatase PP1-arch2 was identified, cloned, and sequences from the methanogenic archaeon Methanosarcina thermophila TM-1. According to the authors, the activity of the recombinant PP1-arch2 was sensitive to several naturally occurring microbial toxins known to potently inhibit eucaryal PP1 and PP2A, including microcystin-LR, okadaic acid, tautomycin, and calyculin A.

Serine/threonine protein phosphatases, such as PP1, have also been reviewed by Wera and Hemmings (1995 Biochem J 311: 17-29). These authors say "Based on biochemical parameters, Ser/Thr protein phosphatases were initially divided into two classes: type-1 phosphatases (PP1) are inhibited by two heat-stable proteins, termed inhibitor-1 (I-1) and inhibitor-2 (I-2), and preferentially dephosphorylate the β-subunit of phosphorylase kinase, whereas type-2 phosphatases are insensitive to the heat-stable inhibitors and preferentially dephosphorylate the α-subunit of phosphorylase kinase. Type-2 phosphatases can be further subdivided into spontaneously active (PP2A), Ca²⁺-dependent (PP2B) and Mg²⁺-dependent (PP2C) classes. Molecular cloning revealed that PP2A was in fact much more closely related to PP1 than to PP2C. Moreover, in the past few years many novel protein phosphatases have been identified that do not fit into the above classification. Many of these protein phosphatases are closely related to the existing classes or are intermediates between classes. From a phylogenetic point of view, it would be more reasonable to group PP1, PP2A and PP2B in a family I and PP2C in a family II. Recently a detailed comparison of the primary structures of 44 different protein Ser/Thr phosphatases, excluding PP2C, was carried out. This revealed a common core structure that comprises two domains. The first domain is predicted to fold as a single β sheet flanked by α helices, whereas the second is predominantly helical. This multiple alignment also shows that family I can be subdivided into PP1-like, PP2A-like and PP2B-like enzymes. Several recent reviews deal with the structure and function of PP1. The catalytic subunit of PP1, termed PPlc, was originally isolated as a 33 kDa protein, but this turned out to be a proteolytic fragment of a 37 kDa protein. PP1c has been crystallized in the presence of the phosphatase inhibitor microcystin. Initially two isoforms of PP1c were cloned, termed α and β. PP1cβ was later shown to be a cloning artefact. In rat, cDNA cloning revealed the existence of at least four isoforms, termed α, γ1, γ2 and δ. Interestingly, the γ1 and γ2 isoforms are produced by alternative splicing of the same primary transcript. The mouse homologues, derived from a brain library, were termed dis2m1 (for the γ1 isoform) and dis2m2 (for the δ isoform). Later a homologue of the δ isoform was found in a rabbit cDNA library and termed β. Furthermore PP1c can associate with proteins that inhibit its activity. These inhibitors can also be considered as regulatory subunits. The holoenzymes are named according to their apparent subcellular location."

Further teachings on serine/threonine protein phosphatases, such as PP1, can be found in the references cited by Wera and Hemmings (*ibid*).

PP1 has some interesting properties that can be utilised in the present invention. For example, and if necessary with appropriate linkers, PP1 can be bound to solid supports. By way of example, PP1 can be bound to Sepharose columns in the assay methods of the present invention. In this respect, the teachings of Zhao S, Lee EY *(Biochemistry* 1997 Jul8; 36(27):8318-8324) can be adapted. Here, the authors demonstrated that the catalytic subunit of rabbit muscle protein phosphatase-1 (PP1) binds to muscle phosphofructokinase (6-phosphofructo-1-kinase, PFK). In those studies, a protein of 85 kDa was isolated from rat muscle by affinity chromatography on PP1-Sephraose and was identified as phosphofructokinase by partial amino acid sequence analysis.

PP1 can also be phosphorylated. In this regard, Villa-Moruzzi E, Puntoni F *(Biochem Biophys Res Commun* 1996Feb27;219(3):863-867) report that phosphatase-1 (PP1) is phosphorylated *"in vitro"* by the tyrosine-kinases c-src, v-src and v-abl. In the case of src, the authors report that this induces enzyme inactivation.

A number of very potent and specific natural toxins of serine/threonine phosphatases (such as those comprising as the catalytic domain PP1α) have been discovered, and its structure, complexed with one of these toxins (microcystin), was recently elucidated to 2.1 Å. The active site is highly featured and we believe that the surface grooves of PP1 are promising from the viewpoint of discovering true small molecule selective inhibitors of PP-1 by high through put screening (HTS).

A paper describing the interaction between PKR and PP1α is Szyszka *et al.,* 1989, J. Biol. Chem. 264: 3827-3831. However, that paper does not suggest a possible use of that interaction in a screen for putative anti-viral agents.

Like the teachings of US-A-579713, He *et al* (1997 PNAS 94: 843-848) report that the γ₁34.5 protein of herpes simplex virus 1 complexes with protein phosphatase 1α to dephosphorylate the α subunit of the eukaryotic translation initiation factor 2 and preclude the shutoff of protein synthesis by double-stranded RNA-activated protein kinase. Here the authors say that okadaic acid inhibits PP1 activity. However, the paper does not suggest a screen for putative anti-viral agents according to the present invention. In contrast to the teachings of He *et al (ibid),* Wera and Hemmings (1995 Biochem J 311: 17-29) state that okadaic acid activates PP1 activity (see the legend to Figure 1 thereof). Likewise, that paper does not suggest a screen for putative anti-viral agents according to the present invention.

As indicated, preferably the serine/threonine phosphatase is the catalytic domain PP1α.of a serine/threonine phosphatase and/or the targeting sub-unit normally associated with PP1α.

Preferred nucleotide and amino acid sequences for PP1α are presented as SEQ ID No. 1 and SEQ ID No. 2 respectively. These sequences have been taken from MEDLINE reference 93209540 and are derived from Durfee, T., Becherer, K., Chen, P.L., Yeh, S.H., Yang, Y., Kilburn, A.E., Lee, W.H. and Elledge, S.J. "The retinoblastoma protein associates with the protein phosphatase type 1 catalytic subunit", Genes Dev. 7 (4), 555-569 (1993) (Fig. 5).

Nucleotide and amino acid sequences for one of the targeting sub-units normally associated with PP1α are presented as SEQ ID No. 3 and SEQ ID No. 4 respectively.

As indicated above, a preferred screen of the present invention is a screen for agents that can inhibit the modulation (such as deactivation) of PKR-P by PP1α and/or the targeting sub-unit normally associated with PP1α. Such an agent should increase the concentration of PKR-P and thus force cell death.

In accordance with the present invention, the serine/threonine phosphatase (preferably that comprising as the catalytic domain PP1α and/or the targeting sub-unit normally associated with PP1α) could be associated with (such as bound to and/or complexed with) one or more host components and/or one or more viral components.

Thus, we believe that the interaction between protein phosphatase PP1α and/or the targeting sub-unit normally associated with PP1α and the ds-RNA-activated Protein Kinase (PFR) is a potential target for the development of broad spectrum antiviral therapy (e.g. HCV, Flu, Herpes). In addition, targeting the interaction between PP1 and their substrates constitutes a novel approach that can open the way to PP1 as being a new target family for small molecule intervention, with important implications for a large variety of therapeutic areas (including cardiovascular and tissue repair).

We believe that supporting evidence that the PKR pathway plays a major role in an antiviral response comes from the fact that a considerable number of viruses (see Table 1) have evolved one or more mechanisms to interfere with this pathway. Moreover, PolyI.rC (a synthetic dsRNA)/IFNγ protection of mice against ECMV infection does not occur with pkr⁻/pkr⁻ transgenic mice (Yang, Reis *et al.* 1995).

Thus, now, viral inhibitors of the PKR pathway can be broadly grouped into three categories:
1) protein inhibitors of PKR which either degrade PKR (e.g. poliovirus activated PKR protease) or prevent its interaction with eIF2a (e.g. HCV and most other viruses);
2) RNA inhibitors of PKR which prevent activation of PKR by ds-RNA;
3) activators of protein phosphatase, especially the catalytic domain PP1α.

With this in mind, there are now three classes of potential anti-viral agents - these being modulators of those same viral inhibitors of the PKR pathway. For convenience, these anti-viral agents can be classified as being:
1) modulators of protein inhibitors of PKR which either degrade PKR or prevent its interaction with eIF2a;
2) modulators of RNA inhibitors of PKR which prevent activation of PKR by ds-RNA;
3) modulators of activators of PP1α and/or the targeting sub-unit normally associated with PP1α .

The third category is that of the present invention. Nevertheless, the present invention also encompasses combination therapies wherein one or more of these modulators may be used but wherein at least one of those modulators is a modulator of an activator of PP1α and/or the targeting sub-unit normally associated with PP1α .

Another screen encompassed by the present invention is a screen for agents that can inhibit the modulation of eIF2α-P by PP1α and/or the targeting sub-unit normally associated with PP1α . Here, the targeting sub-unit normally associated with PP1α could be GADD34. Such an agent should also force cell death.

We believe that the assay of the present invention would be useful for screening for at least agents capable of affecting any one or more of HIV, HCV, Vaccinia, HSV-1, Influenza, Reoviruses, Rotaviruses, Epstein-Barr Virus, Adenovirus, Poliovirus, VSV activity.

Hence, preferably the assay of the present invention is used to screen for agents capable of affecting any one or more of HIV, HCV, Vaccinia, HSV-1, Influenza, Reoviruses, Rotaviruses, Epstein-Barr Virus, Adenovirus, Poliovirus, VSV activity.

More preferably the assay of the present invention is used to screen for agents capable of affecting any one or more of HIV, HCV, Vaccinia, Influenza, Reoviruses, Rotaviruses, Epstein-Barr Virus, Adenovirus, Poliovirus, VSV activity.

The anti-viral agent of the present invention can be generically called a "PP1α inhibitor". Here, the inhibitorsmay interact with or affect PP1α and/or the targeting sub-unit normally associated with PP1α.

The PP1α inhibitor may be used with other therapeutics. By way of example, and in the case of HIV-1, the PP1α inhibitor could be added to available combination therapies, including nucleoside, non-nucleoside and protease inhibitors. In the case of HCV, the PP1α inhibitor could be added to available therapies, including protease inhibitors.

We also realise that in some cases, such as vaccinia or influenza, mechanisms have evolved which minimize the extent of phosphorylation of eIF2α. However, even though these viruses are not expected to achieve complete inhibition of eIF2α phosphorylation, it should still nevertheless be possible to accumulate a sufficient amount of the inactive form of the translation initiation factor.

Thus, the assay of the present invention provides a means to discover PP1α inhibitors, such as novel PP1α inhibitors, for use as antiviral therapeutics.

A suitable assay according to the present invention may comprise use of the relatively simple colorimetric (or even filter binding) assay (compatible with automation) that has been recently developed for this enzyme (see Gupta, Ogawa *et al.* 1997).

Potential assay formats include any one or more of the following:
a) a generic method for screening for antiviral agents that prevent virus mediated inhibition of translation shut-down comprising the steps of: incubating the catalytic subunit PP1α and optionally the associated regulatory subunit with PKR or eIF2α (in its phosphorylated and/or unphosphorylated form) and the agent, and determining whether the agent can interfere with the interaction between PP1α (and preferably the regulatory subunit) and PKR or eIF2α by one of a variety of methods including: (1) Enzyme Linked Immunosorbent Assay (ELISA; Engvall E. et al., *Immunochemistry* 8: 871-874), (2) Homogeneous Time Resolved Fluorescence (HTRF; Yamanaka et al., *High Throughput Screening,* 1997, 377-388, Ed Devlin JP, New York), (3) Scintillation Proximity Assay (SPA; Amersham Pharmacia Biotech).
b) incubating the catalytic subunit PP1α and preferably its associated regulatory subunit with radio-phosphorylated PKR or eIF2α and the agent, and determining whether the agent prevents the PP1α-dependent de-phosphorylation of PKR or eIF2α by measuring the amount of radioactivity bound to membranes capable of binding proteins.
c) incubating cells infected with any of the virus mentioned above, with the agent and measuring the effect of the agent on the ability of the virus to replicate as measured by any of a variety of techniques, including plaque assay, nucleic acid replication, protein synthesis; the same type of assay could be carried out with appropriate surrogate systems where culturing the virus may prove difficult.

In accordance with the present invention it is also possible to observe the translational or transcriptional activities of the sequences coding for the phosphatase of the present invention, preferably PP1 (more preferably a phosphatase comprising the catalytic subunit PP1α and/or the targeting sub-unit normally associated with PP1α), or the sequences controlling the expression thereof, in a suitable host cell and then to see if agents can modulate such activities. Thus, and by way of example, the present invention also includes a method which comprises determining whether an agent has an effect on the translational machinery of an infected host cell or an uninfected viral host cell.

The present invention also encompasses combinations of the agents of the present invention.

In addition, the present invention encompasses combinations comprising one or more of the agents of the present invention and other therapeutic agents - which agents may be administered simultaneously, sequentially, previously or subsequently in relationship to the agents of the present invention. By way of example, typical combination therapies include administration of a PP1α inhibitor and an inhibitor of NS5A and/or interferon.

Here the term "interferon" is used in the normal sense in the art. For example, reference may be made to Oxford Dictionary of Biochemistry and Molecular Biology (Oxford University Press, 1997) - *viz.* "Interferon *abbr.:* IFN; any member of a group of proteins that form a closely related group of nonviral proteins, of *M*ᵣ in the range 15,000 to 30,000, that are produced and liberated by animal cells following exposure to a variety of inducing agents; they are not normally present in uninduced cells. They exert nonspecific, antiviral activity, at least in homologous cells, through cellular metabolic processes involving the synthesis of both RNA and protein. Viruses are the most potent inducing agents, but interferons can also be induced by exposure of cells to a wide variety of microorganisms, bacterial endotoxins, phytohemagglutinins, and other substances. Interferons do not prevent viral penetration into cells but induce within the cells a complex of inhibitors of protein synthesis that block translation of viral mRNA, but do not the cell's own mRNA. Among the interferon inhibitors are a protein kinase, which phosphorylates an initiation factor in protein synthesis, and an oligonucleotide synthetase, which catalyses the synthesis of pppA2'p5'A2'p5'A *(abbr.:* 2,5-A) - this trinucleotide activates a latent cellular endonuclease that degrades the viral mRNA. Interferons are classified into three types. IFN-α (*formerly* leukocyte interferon) comprises many examples; they are produced by macrophages, have antiviral activities, and stimulate production of two enzymes: a protein kinase and an oligoadenylate synthetase; they exist as monomers. Example, (human) α-4B precursor: database code INA4_HUMAN, 189 amino acids (21.78 kDa). IFN-β *(formerly* fibroblast interferon) has antiviral, antibacterial, and anticancer activities. It is related to the α family (three motifs). Example (human, precursor): database code INB_HUMAN, 187 amino acids (22,27 kDa). IFN-γ (*formerly* immune interferon) is produced by lymphocytes activated by specific antigens or mitogens. In addition to antiviral activity it has important immunoregulatory functions: it is a potent activator of macrophages, it has antiproliferative effects on transformed cells, and can potentiate the antiviral and antitumour effects of the type I interferons (α and β). It is a glycoprotein and exists as a homodimer. Example (human, precursor): database code ING_HUMAN, 166 amino acids (19.33 kDa). Interferons are produced commercially either by *in vitro* culture of human leukocytes or by recombinant DNA or RNA technology, γ interferon requiring a system that effects glycosylation."

Without wishing to be bound by theory, we believe that combining phosphatase inhibitors with IFN could increase the efficacy of IFN treatment

The present invention is advantageous because it offers means of identifying targets based on essential functions for identifying potential viral inhibitors; it provides means for screening agents with a view to identifying anti-viral targets; it enables the identification of a nucleotide sequence and its EP thereof which are distinct from nucleotide sequences and EPs targeted by existing antiviral agents. Such a nucleotide sequence and EP thereof therefore serves as targets for therapeutics based on mechanisms which are likely to be distinct from the mechanisms of existing drugs. In addition, the present invention enables the identification of agents which inhibit the function of the nucleotide sequence and EP thereof by methods disclosed herein and which are distinct from known agents.

In accordance with the present invention it is possible to use coding sequences or EP sequences that have few or no close homologues. Consequently, it is believed that the therapeutic agents directed towards targets (such as genes or gene products) may have fewer side effects and may also have an effective therapeutic effect at a lower overall dosage.

Thus, the present invention relates to certain host nucleic acid and amino acid sequences which may be essential for the pathogenic virus to maintain host cell viability. The present invention also relates to the use of the nucleic acid and amino acid sequences in the diagnosis and treatment of disease. The present invention also relates to the use of the nucleic acid and amino acid sequences to evaluate and/or to screen for agents that can modulate the expression of the nucleotide sequence or the activity of its EP. The present invention further relates to genetically engineered host cells that comprise or express the nucleic acid and amino acid sequences to evaluate and/or to screen for agents that can modulate the EP of the nucleotide sequence

The term "nucleotide sequence" as used herein refers to an oligonucleotide sequence or polynucleotide sequence, and variants, homologues, fragments and derivatives thereof (such as portions thereof). The nucleotide sequence may be DNA or RNA of genomic or synthetic or recombinant origin which may be double-stranded or single-stranded whether representing the sense or antisense strand. Preferably, the term nucleotide sequence is prepared by use of recombinant DNA techniques (e.g. recombinant DNA).

Preferably, the term "nucleotide sequence" means RNA.

In a preferred embodiment, the present invention does not cover the native nucleotide coding sequence according to the present invention in its natural environment when it is under the control of its native promoter which is also in its natural environment. For ease of reference, we shall call this preferred embodiment the "non-native nucleotide sequence".

As used herein "amino acid sequence" refers to peptide, polypeptide or protein sequences or portions thereof.

In a preferred embodiment, the present invention does not cover the native EP according to the present invention when it is in its natural environment and when it has been expressed by its native nucleotide coding sequence which is also in its natural environment and when that nucleotide sequence is under the control of its native promoter which is also in its natural environment. For ease of reference, we shall call this preferred embodiment the "non-native amino acid sequence".

As used herein, the term "the EP" refers to an EP as such, as well as fusion protein derivatives thereof.

The EP of the present invention may be the same as the naturally occuring form - for this aspect, preferably the EP has a non-native amino acid sequence - or is a variant, homologue, fragment or derivative thereof. In addition, or in the alternative, the EP is the isolated EP and/or the purified EP. The EP can be obtainable from or produced by any suitable source, whether natural or not, or it may be synthetic, semi-synthetic or recombinant.

The EP coding sequence may be the same as the naturally occuring form - for this aspect, preferably the EP coding sequence is the non-native nucleotide sequence - or is a variant, homologue, fragment or derivative thereof. In addition, or in the alternative, the EP coding sequence is an isolated EP coding sequence and/or a purified EP coding sequence. The EP coding sequence can be obtainable from or produced by any suitable source, whether natural or not, or it may be synthetic, semi-synthetic or recombinant.

Altered EP polynucleotide sequences which may be used in accordance with the invention include deletions, insertions or substitutions of different nucleotide residues resulting in a polynucleotide that encodes the same or a functionally equivalent to the EP.

As used herein a "deletion" is defined as a change in either nucleotide or amino acid sequence in which one or more nucleotides or amino acid residues, respectively, are absent.

As used herein an "insertion" or "addition" is that change in a nucleotide or amino acid sequence which has resulted in the addition of one or more nucleotides or amino acid residues, respectively, as compared to the naturally occurring EP.

As used herein "substitution" results from the replacement of one or more nucleotides or amino acids by different nucleotides or amino acids, respectively.

As used herein "naturally occurring" refers to an EP with an amino acid sequence found in nature.

As used herein "biologically active" refers to an EP having structural, regulatory or biochemical functions of the naturally occurring EP.

As used herein, "immunological activity"· is defined as the capability of the natural, recombinant or synthetic EP or any oligopeptide thereof, to induce a specific immune response in appropriate animals or cells and to bind with specific antibodies.

The term "derivative" as used herein includes chemical modification of an EP. Illustrative of such modifications would be replacement of hydrogen by an alkyl, acyl, or amino group.

In one embodiment, the nucleotide sequence and/or the EP thereof are isolated and purified.

As used herein, the terms "isolated" and "purified" refer to molecules, either nucleic or amino acid sequences, that are removed from their natural environment and/or isolated or separated from at least one other component with which they are naturally associated.

The terms "variant", "homologue" or "fragment" in relation to the amino acid sequence for the EP of the present invention include any substitution of, variation of, modification of, replacement of, deletion of or addition of one (or more) amino acid from or to the sequence providing the resultant EP has an activity, preferably being at least as biologically active as the polypeptide shown as SEQ ID No. 2. In particular, the term "homologue" covers homology with respect to structure and/or function. With respect to sequence homology, preferably there is at least 75%, more preferably at least 85 %, more preferably at least 90% homology to the sequence shown as SEQ ID No. 2. More preferably there is at least 95%, more preferably at least 98%, homology to the sequence shown as SEQ ID No. 2.

The terms "variant", "homologue" or "fragment" in relation to the nucleotide sequence coding for the EP of the present invention include any substitution of, variation of, modification of, replacement of, deletion of or addition of one (or more) nucleic acid from or to the sequence providing the resultant nucleotide sequence codes for or is capable of coding for an EP preferably being at least as biologically active as the EP encoded by the sequences shown as SEQ ID No.1. In particular, the term "homologue" covers homology with respect to structure and/or function providing the resultant nucleotide sequence codes for or is capable of coding for an EP being at least as biologically active as the EP encoded by the sequences shown as SEQ ID No.1. With respect to sequence homology, preferably there is at least 75%, more preferably at least 85%, more preferably at least 90% homology to the sequence shown as SEQ ID No.1. More preferably there is at least 95%, more preferably at least 98%, homology to the sequence shown as SEQ ID No. 1.

The terms "variant", "homologue" or "fragment" in relation to the amino acid sequence for the EP of the present invention include any substitution of, variation of, modification of, replacement of, deletion of or addition of one (or more) amino acid from or to the sequence providing the resultant EP has an activity, preferably being at least as biologically active as the polypeptide shown as SEQ ID No. 4. In particular, the term "homologue" covers homology with respect to structure and/or function. With respect to sequence homology, preferably there is at least 75%, more preferably at least 85%, more preferably at least 90% homology to the sequence shown as SEQ ID No. 4. More preferably there is at least 95%, more preferably at least 98%, homology to the sequence shown as SEQ ID No. 4.

The terms "variant", "homologue" or "fragment" in relation to the nucleotide sequence coding for the EP of the present invention include any substitution of, variation of, modification of, replacement of, deletion of or addition of one (or more) nucleic acid from or to the sequence providing the resultant nucleotide sequence codes for or is capable of coding for an EP preferably being at least as biologically active as the EP encoded by the sequences shown as SEQ ID No. 3. In particular, the term "homologue" covers homology with respect to structure and/or function providing the resultant nucleotide sequence codes for or is capable of coding for an EP being at least as biologically active as the EP encoded by the sequences shown as SEQ ID No. 3. With respect to sequence homology, preferably there is at least 75%, more preferably at least 85%, more preferably at least 90% homology to the sequence shown as SEQ ID No. 3. More preferably there is at least 95 %, more preferably at least 98%, homology to the sequence shown as SEQ ID No. 3.

In particular, the term "homology" as used herein may be equated with the term "identity". Relative sequence homology (i.e. sequence identity) can be determined by commercially available computer programs that can calculate % homology between two or more sequences. A typical example of such a computer program is CLUSTAL.

"Substantial homology", where homology indicates sequence identity, means more than 80% sequence identity, preferably more than 85% sequence identity and most preferably a sequence identity of 90% or more, as judged by direct sequence alignment and comparison.

Sequence homology (or identity) may moreover be determined using any suitable homology algorithm, using for example default parameters. Advantageously, the BLAST algorithm is employed, with parameters set to default values. The BLAST algorithm is described in detail at http://www.ncbi.nih.gov/BLAST/blast_help.html, which is incorporated herein by reference. The search parameters are defined as follows, and are advantageously set to the defined default parameters.

Advantageously, "substantial homology" when assessed by BLAST equates to sequences which match with an EXPECT value of at least about 7, preferably at least about 9 and most preferably 10 or more. The default threshold for EXPECT in BLAST searching is usually 10.

BLAST (Basic Local Alignment Search Tool) is the heuristic search algorithm employed by the programs blastp, blastn, blastx, tblastn, and tblastx; these programs ascribe significance to their findings using the statistical methods of Karlin and Altschul (see http://www.ncbi.nih.gov/BLAST/blast_help.htm1) with a few enhancements. The BLAST programs were tailored for sequence similarity searching, for example to identify homologues to a query sequence. The programs are not generally useful for motif-style searching. For a discussion of basic issues in similarity searching of sequence databases, see Altschul *et al* (1994) Nature Genetics 6:119-129.

The five BLAST programs available at http://www.ncbi.nlm.nih.gov perform the following tasks:
**blastp** compares an amino acid query sequence against a protein sequence database;
**blastn** compares a nucleotide query sequence against a nucleotide sequence database;
**blastx** compares the six-frame conceptual translation products of a nucleotide query sequence (both strands) against a protein sequence database;
**tblastn** compares a protein query sequence against a nucleotide sequence database dynamically translated in all six reading frames (both strands).
**tblastx** compares the six-frame translations of a nucleotide query sequence against the six-frame translations of a nucleotide sequence database.

BLAST uses the following search parameters:

HISTOGRAM Display a histogram of scores for each search; default is yes. (See parameter H in the BLAST Manual).

DESCRIPTION Restricts the number of short descriptions of matching sequences reported to the number specified; default limit is 100 descriptions. (See parameter V in the manual page). See also EXPECT and CUTOFF.

ALIGNMENTS Restricts database sequences to the number specified for which high-scoring segment pairs (HSPs) are reported; the default limit is 50. If more database sequences than this happen to satisfy the statistical significance threshold for reporting (see EXPECT and CUTOFF below), only the matches ascribed the greatest statistical significance are reported. (See parameter B in the BLAST Manual).

EXPECT The statistical significance threshold for reporting matches against database sequences; the default value is 10, such that 10 matches are expected to be found merely by chance, according to the stochastic model of Karlin and Altschul (1990). If the statistical significance ascribed to a match is greater than the EXPECT threshold, the match will not be reported. Lower EXPECT thresholds are more stringent, leading to fewer chance matches being reported. Fractional values are acceptable. (See parameter E in the BLAST Manual).

CUTOFF Cutoff score for reporting high-scoring segment pairs. The default value is calculated from the EXPECT value (see above). HSPs are reported for a database sequence only if the statistical significance ascribed to them is at least as high as would be ascribed to a lone HSP having a score equal to the CUTOFF value. Higher CUTOFF values are more stringent, leading to fewer chance matches being reported. (See parameter S in the BLAST Manual). Typically, significance thresholds can be more intuitively managed using EXPECT.

MATRIX Specify an alternate scoring matrix for BLASTP, BLASTX, TBLASTN and TBLASTX. The default matrix is BLOSUM62 (Henikoff & Henikoff, 1992). The valid alternative choices include: PAM40, PAM120, PAM250 and IDENTITY. No alternate scoring matrices are available for BLASTN; specifying the MATRIX directive in BLASTN requests returns an error response.

STRAND Restrict a TBLASTN search to just the top or bottom strand of the database sequences; or restrict a BLASTN, BLASTX or TBLASTX search to just reading frames on the top or bottom strand of the query sequence.

FILTER Mask off segments of the query sequence that have low compositional complexity, as determined by the SEG program of Wootton & Federhen (1993) Computers and Chemistry 17:149-163, or segments consisting of short-periodicity internal repeats, as determined by the XNU program of Claverie & States (1993) Computers and Chemistry 17:191-201, or, for BLASTN, by the DUST program of Tatusov and Lipman (see http://www.ncbi.nlm.nih.gov). Filtering can eliminate statistically significant but biologically uninteresting reports from the blast output (e.g., hits against common acidic-, basic- or proline-rich regions), leaving the more biologically interesting regions of the query sequence available for specific matching against database sequences.

Low complexity sequence found by a filter program is substituted using the letter "N" in nucleotide sequence (e.g., "NNNNNNNNNNNNN") and the letter "X" in protein sequences (e.g., "XXXXXXXXX").

Filtering is only applied to the query sequence (or its translation products), not to database sequences. Default filtering is DUST for BLASTN, SEG for other programs.

It is not unusual for nothing at all to be masked by SEG, XNU, or both, when applied to sequences in SWISS-PROT, so filtering should not be expected to always yield an effect. Furthermore, in some cases, sequences are masked in their entirety, indicating that the statistical significance of any matches reported against the unfiltered query sequence should be suspect.

NCBI-gi Causes NCBI gi identifiers to be shown in the output, in addition to the accession and/or locus name.

Most preferably, sequence comparisons are conducted using the simple BLAST search algorithm provided at http://www.ncbi.nlm.nih.gov/BLAST.

Other computer program methods to determine identify and similarity between the two sequences include but are not limited to the GCG program package (Devereux *et al* 1984 Nucleic Acids Research 12: 387and FASTA (Atschul *et al* 1990 J Molec Biol 403-410).

As used herein, the terms "variant", "homologue", "fragment" and "deriavtive" are synonymous with allelic variations of the sequences.

The term "variant" also encompasses sequences that are complementary to sequences that are capable of hydridising to the nucleotide sequences presented herein.

Preferably, the term "variant" encompasses sequences that are complementary to sequences that are capable of hydridising under stringent conditions (eg. 65°C and 0.1xSSC {1xSSC = 0.15 M NaCl, 0.015 Na₃ citrate pH 7.0}) to the nucleotide sequences presented herein.

The present invention also relates to nucleotide sequences that can hybridise to the nucleotide sequences of the present invention (including complementary sequences of those presented herein).

The present invention also relates to nucleotide sequences that are complementary to the sequences that can hybridise to the nucleotide sequences of the present invention (including complementary sequences of those presented herein).

The term "hybridization" as used herein shall include "the process by which a strand of nucleic acid joins with a complementary strand through base pairing" (Coombs J (1994) Dictionary of Biotechnology, Stockton Press, New York NY) as well as the process of amplification as carried out in polymerase chain reaction technologies as described in Dieffenbach CW and GS Dveksler (1995, PCR Primer, a Laboratory Manual, Cold Spring Harbor Press, Plainview NY).

Also included within the scope of the present invention are nucleotide sequences that are capable of hybridizing to the nucleotide sequence of SEQ ID No.1 (and/or or SEQ ID No.3) or other sequences coding for the amino acid sequence of SEQ ID No. 2 (and/or SEQ ID No.4, respectively) under conditions of intermediate to maximal stringency. Hybridization conditions are based on the melting temperature (Tm) of the nucleic acid binding complex, as taught in Berger and Kimmel (1987, Guide to Molecular Cloning Techniques, Methods in Enzymology, Vol 152, Academic Press, San Diego CA), and confer a defined "stringency" as explained below.

Maximum stringency typically occurs at about Tm-5°C (5°C below the Tm of the probe); high stringency at about 5°C to 10°C below Tm; intermediate stringency at about 10°C to 20°C below Tm; and low stringency at about 20°C to 25°C below Tm. As will be understood by those of skill in the art, a maximum stringency hybridization can be used to identify or detect identical polynucleotide sequences while an intermediate (or low) stringency hybridization can be used to identify or detect similar or related polynucleotide sequences.

In a preferred aspect, the present invention covers nucleotide sequences that can hybridise to the nucleotide sequence of the present invention under stringent conditions (e.g. 65°C and 0.1xSSC).

The presence of the EP polynucleotide coding sequence can be detected by DNA-DNA or DNA-RNA hybridization or amplification using probes, portions or fragments of the sequence presented as SEQ ID No. 1 (and/or SEQ ID No.3) or other sequences coding for the amino acid sequence of SEQ ID No. 2 (and/or SEQ ID No.4, respectively). Nucleic acid amplification based assays involve the use of oligonucleotides or oligomers based on the EP coding sequence to detect transformants containing the EP DNA or RNA.

As used herein "oligonucleotides" or "oligomers" may refer to a nucleic acid sequence of at least about 10 nucleotides and as many as about 60 nucleotides, preferably about 15 to 30 nucleotides, and more preferably about 20-25 nucleotides which can be used as a probe or amplifier. Preferably, oligonucleotides are derived from the 3' region of the nucleotide sequence shown as SEQ ID No. 1 (and/or SEQ ID No.3) or other sequences coding for the amino acid sequence of SEQ ID No. 2 (and/or SEQ ID No.4, respectively).

The term "vector" includes expression vectors and transformation vectors.

The term "expression vector" means a construct capable of *in vivo* or *in vitro* expression.

The term "transformation vector" means a construct capable of being transferred from one species to another.

Thus, the present invention relates to an EP and to a nucleic acid sequence encoding same.

Either or both of the nucleotide sequence coding for the EP or the EP itself may be used to screen for agents that can affect the EP activity. In particular, the nucleotide sequence coding for the EP or the EP itself may be used to screen for agents that can inhibit the EP activity. In addition, the nucleotide sequence coding for the EP or the EP itself may be used to screen for agents that selectively affect the EP activity, such as selectively inhibit the EP activity.

Furthermore, the nucleotide sequence coding for the EP or a sequence that is complementary thereto may also be used in assays in the presence of EP coding sequences from human cells. These competitive assays may provide information regarding the selectivity of an EP inhibitor.

The present invention also covers antibodies to the EP (including a derivative, fragment, homologue or variant thereof). The antibodies to the EP may be used in assays to detect the presence of the EP in human cells. These assays would provide information regarding the tissue distribution of the EP and its biological relevance with respect to particular disease states.

In particular, any one or more of the EP, the nucleotide sequence coding for same, the nucleotide sequences that are complementary to same, and the antibodies directed to same may be used in assays to screen for agents that selectively affect the EP. These assays would provide information regarding the tissue distribution of the EP and to provide information regarding the biological relevance of the EP with respect to particular disease states. These assays would also allow workers to test for and identify agents that are useful to affect the expression of or activity of the EP - such as in a particular tissue or in a particular disease state.

The present invention also relates to polypeptides produced by expression in a host cell into which has been incorporated the foregoing DNA sequences or allelic variations thereof.

The present invention also provides a method for producing a polypeptide having the EP activity, the method comprising the steps of a) transforming a host cell with a nucleotide sequence shown as SEQ ID No. 1 and/or SEQ ID No.3 or a derivative, homologue, variant or fragment thereof; b) culturing the transformed host cell under conditions suitable for the expression of said polypeptide; and c) recovering said polypeptide from the host cell culture.

We believe that inhibiting the activity of the EP is likely to affect essential viral functions. Thus, the EP and/or its coding sequence and/or a sequence capable of hybridising thereto and/or a sequence that are complementary to the sequences capable of hybridising thereto is/are useful for screening drug candidates useful as antiviral. In addition, it is believed that the EP and/or its coding sequence and/or a sequence capable of hybridising thereto sequence that are complementary to the sequences capable of hybridising thereto is/are useful for screening drug candidates for the treatment of viral infections. Also, the EP and/or its coding sequence and/or a sequence capable of hybridising thereto sequence that are complementary to the sequences capable of hybridising thereto is/are useful for testing the selectivity of drug candidates between different the EPs.

As indicated, the present invention relates to a DNA sequence (preferably a cDNA sequence) encoding the EP. In particular, the present invention relates to cDNA sequences encoding the EP.

Included within the scope of the present invention are alleles of the EP. As used herein, an "allele" or "allelic sequence" is an alternative form of the EP. Alleles result from a mutation, i.e., a change in the nucleic acid sequence, and generally produce altered mRNAs or polypeptides whose structure or function may or may not be altered. Any given gene may have none, one or many allelic forms. Common mutational changes which give rise to alleles are generally ascribed to deletions, additions or substitutions of amino acids. Each of these types of changes may occur alone, or in combination with the others, one or more times in a given sequence.

The present invention also relates to DNA segments comprising the DNA sequence of SEQ ID No. 1 and/or SEQ ID No.3 or allelic variations of such sequences.

A highly preferred aspect of the present invention relates to a polypeptide comprising the amino acid sequence of SEQ ID No. 2 and/or SEQ ID No.4. For example, the present invention relates to an isolated polypeptide comprising the amino acid sequence of SEQ ID No. 2 and/or SEQ ID No.4.

The present invention also relates to DNA comprising the DNA sequence of SEQ ID No. 1 and/or SEQ ID No.3, or an allelic variation thereof.

The present invention also relates to non-native DNA comprising the DNA sequence of SEQ ID No. 1 and/or SEQ ID No.3, or an allelic variation thereof.

A highly preferred aspect of the present invention relates to recombinant DNA comprising the DNA sequence of SEQ ID No. 1 or an allelic variation thereof.

The present invention also relates to an assay method for detecting the presence of the EP in cells (such as human cells) comprising: (a) performing a reverse transcriptase-polymerase chain reaction on RNA (such as total RNA) from such cells using a pair of polymerase chain reaction primers that are specific for the EP, as determined from the DNA sequence of SEQ ID No. 1 and/or SEQ ID No.3, or an allelic variation thereof; and (b) assaying the appearance of an appropriately sized PCR (polymerase chain reaction) fragment - such as by agarose gel electrophoresis.

The present invention also relates to a method of identifying agents (such as compounds, other substances or compositions comprising same) that affect (such as inhibit or otherwise modify) the activity of the EP, the method comprising contacting the EP or the nucleotide sequence coding for same with the agent and then measuring the activity of the EP.

The present invention also relates to a method of identifying agents (such as compounds, other substances or compositions comprising same) that selectively affect (such as inhibit or otherwise modify) the activity of the EP, the method comprising contacting the EP or the nucleotide sequence coding for same with the agent and then measuring the activity of the EP.

The present invention also relates to a method of identifying agents (such as compounds, other substances or compositions comprising same) that affect (such as inhibit or otherwise modify) the activity of the EP, the method comprising measuring the activity of the EP in the presence of the agent or after the addition of the agent in: (a) a cell line into which has been incorporated recombinant construct comprising the nucleotide sequence of SEQ ID No. 1 and/or SEQ ID No.3, or an allelic variation thereof, or (b) a cell population or cell line that naturally selectively expresses the EP and then measuring the activity of the EP.

The present invention also relates to a method of identifying agents (such as compounds, other substances or compositions comprising same) that selectively affect (such as inhibit or otherwise modify) the activity of the EP, the method comprising measuring the activity of the EP in the presence of the agent or after the addition of the agent in: (a) a cell line into which has been incorporated recombinant construct comprising the nucleotide sequence of SEQ ID No. 1 and/or SEQ ID No.3, or an allelic variation thereof, or (b) a cell population or cell line that naturally selectively expresses the EP and then measuring the activity of the EP.

Some specific embodiments of the present invention relate to assay methods for the identification of antiviral agents using assays for antiviral agents which may be carried out both in whole cell preparations and in *ex vivo* cell-free systems. In each instance, the assay target is the target nucleotide sequence - which is essential for the virus to maintain cell viability - and/or its EP. Candidate agents which are found to inhibit the target nucleotide sequence and/or the EP in any assay method of the present invention are thus identified as potential antiviral agents. It is expected that the assay methods of the present invention will be suitable for both small and large-scale screening of test compounds as well as in quantitative assays such as serial dilution studies wherein the target nuceotide sequence or its EP thereof are exposed to a range of candidate agent concentrations.

When the assay methods of the present invention are carried out as a whole-cell assay, the target nucleotide sequence and/or its EP and the entire living host cell may be exposed to the candidate agent under conditons normally suitable for growth. Such conditions including essential nutrients, optimal temperatures and other parameters, depend upon the particular viral strain being used and will be well known in the art. Inhibition of the target nucleotide sequence and/or the EP may be determined in a number of ways including observing the cell culture's growth or lack therof. Such observation may be made visually, by optical densitometric or other light absorption/scattering means or by yet other suitable means, whether manual or automated.

In the above whole-cell assay, an observed lack of cell growth may be due to inhibition of the target nucleotide sequence and/or the EP or may be due to an entirely different effect of the candidate agent and further evaluation may be required to establish the mechanism of action and to determine whether the candidate agent is a specific inhibitor of the target. Accordingly and in a preferred embodiment of the present invention, the method may be performed as a paired-cell assay in which each test compound is separately tested against two different host cells, the first host cells having a target with altered properties making it more susceptible to inhibition compared with that of the second host cells.

One manner of achieving differential susceptibility is by using mutant strains expressing a modified target product. A particularly useful strain is one having a temperature sensitive ("ts") mutation as a result of which the target is more prone than the wild type target to loss of functionality at high temperatures (that is, temperatures higher than optimal, but still permitting growth in wild type cells). When grown at semi-permissive temperatures, the activity of a ts mutant target may be attenuated but sufficient for growth.

Alternatively or in conjunction with the above, differential susceptibility to target inhibitors may be obtained by using a second host cell which has altered properties making it less susceptible to inhibition compared with that of a wild type cells as for example, a host cell which has been genetically manipulated to cause overexpression of the target. Such overexpression can be achieved by placing into a wild type cell a plasmid carrying the nucleotide sequence for the target. The techniques for generating temperature sensitive mutants, for preparing specific plasmids and for transforming cell lines with such plasmids are well known in the art.

Alternatively or in conjunction with the above, the access of candidate agents to a cell or an organism, may be enhanced by mutating or deleting a gene or genes which encode a protein or proteins responsible for providing a permeability barrier for a cell or an organism.

The present invention also relates to a method for identifying antiviral agents utilising cell systems that are sensitive to perturbation to one or several transcriptional/translational components.

By way of example, the present invention relates to a method of constructing mutant host cells in which one or more of the transcriptional/translational components is present in an altered form or in a different amount compared with a corresponding wild-type cell. This method further involves examining a candidate agent for its ability to perturb transcriptional/translation by assessing the impact it has on the growth of the mutant and wild-type cells. Agents which perturb transcriptional/translation by acting on a particular component that participates in transcriptional/translation may cause a mutant host cell which has an altered form or amount of that component to grow differently from the corresponding wild-type cell but do not affect the growth relative to the wild type cell of other mutant cells bearing alterations in other components participating in transcription/translation. This method thus provides not only a means to identify whether a candidate agent perturbs transcriptional/translation but also an indication of the site at which it exerts its effects. The transcriptional/translation component which is present in altered form or amount in a cell whose growth is affected by a candidate agent is likely to be the site of action of the agent.

Representative examples of appropriate hosts for use in the assay of the present invention include:
animal cells such as and Bowes melanoma cells, BHK, CHO, HeLa, COS, C127, 3T3, 293 cells
fungal cells, such as yeast cells and *Aspergillus* cells;
bacterial cells, such as *Bacillus subtilis,* streptococci, staphlococci, enterococci *E. coli,* streptomyces;
insect cells such as *Drosophila* S2 and *Spodoptera* Sf9 cells;
plant cells.

Thus, in accordance with the present invention, the sequences of the present invention may be expressed in a yeast cell which is constructed to increase the expression of a suitable marker protein when in the presence of an inhibitor of the expression or activity of the sequences when in the presence of a suitable agent. This aspect of the present invention concerns use of a yeast cell in screening agents effective to inhibit the action of, for example, serine/threonine phosphatase in a host eukaryotic cell. The cell may include an expressable gene encoding a mammalian interferon-induced, double-stranded RNA-activated protein kinase; a suitable reporter sequence; and a sequence coding for serine/threonine phosphatase or an active fragment thereof.

Proteins that may be agents putative agents according to the present invention can also be identified using a yeast genetic system known as the two-hybrid system (Fields & Song, Nature, 340, 245-246, 1989; Chien et al., Proc. Natl. Acad. Sci. USA 88, 9578-9582, 1991). This technique is summarised in US-A-5738985 - which states that the technique requires the availability of a gene or cDNA encoding one of the two proteins which interact with each other. The gene or cDNA is cloned into a specific plasmid in such a way that it is expressed fused to the DNA-binding domain of a yeast transcriptional activator such as GAL4 which has two separable and functionally essential domains, one for DNA-binding and the other for transcriptional activation. In parallel, genes or cDNAs encoding putative binding partners of the known component are cloned in such a way that each putative partner is expressed fused to the transcriptional activation domain of the same DNA-binding protein. Introduction of both types of fusion into the same yeast cell results in generation of functional DNA-binding protein only if the fusion partners of the two domains of this protein interact with one another closely enough to bring together its two separately-expressed domains. Clones which produce such functional DNA-binding protein can be selected very easily by plating them on a medium which requires the yeast to produce an enzyme that is under the control of the DNA-binding protein. The gene or cDNA for the partner which binds to the previously identified component can then be recovered from yeast clones which grow on the selective medium.

US-A-5738985 also reports on other methods that may be adapted for use in the assay method of the present invention. For example, and according to US-A-5738985, it is possible to use the component in question as an affinity ligand to identify viral and cellular products which bind to it; labeling this component with a detectable label and using it as a probe to detect viral and cellular products on blots of electrophoresis gels; labeling the component and using it to probe libraries of viral and cellular genes and/or cDNAs; labeling the component and using it to probe cDNA expression libraries to find clones synthesizing proteins which can bind to the component; performing UV-crosslinking studies to identify viral or cellular products which can bind to the component; using the component in gel retardation assays which would detect its ability to bind to viral or cellular nucleic acids; performing footprinting analyses to identify the regions within a nucleic acid to which the component binds; and so on.

The present invention also provides a method for identifying antiviral agents which interfere with steps in translational accuracy, such as maintaining a proper reading frame during translation and terminating translation at a stop codon. This method involves constructing mutant host cells in which a detectable reporter polypeptide can only be produced if the normal process of staying in one reading frame or of terminating translation at a stop codon has been disrupted. This method further involves contacting the mutant host cells with a candidate agent to examine whether it increases the production of the reporter polypeptide.

The reporter polypeptide can be a peptide which is directly detectable, such as by providing a colorimetric signal, or is indirectly detectable, such as by providing a suitable binding domain for a labelled probe (such as an antibody).

The present invention also provides a method of screening an agent for specific binding affinity with the EP (or a derivative, homologue, variant or fragment thereof) or the nucleotide sequence coding for same (including a derivative, homologue, variant or fragment thereof), the method comprising the steps of: a) providing a candidate agent; b) combining the EP (or the derivative, homologue, variant or fragment thereof) or the nucleotide sequence coding for same (or the derivative, homologue, variant or fragment thereof) with the candidate agent for a time sufficient to allow binding under suitable conditions; such binding or interaction being associated with a second component capable of providing a detectable signal in response to the binding or interaction of the EP or the nucleotide sequence encoding same with the agent and c) determining whether the agent binds to or otherwise interacts with and activates or inhibits an activity of the EP (or the derivative, homologue, variant or fragment thereof) or the nucleotide sequence coding for same (or the derivative, homologue, variant or fragment thereof) by detecting the presence or absence of a signal generated from the binding and/or interaction of the agent with the EP (or the derivative, homologue, variant or fragment thereof) or the nucleotide sequence coding for same (or the derivative, homologue, variant or fragment thereof).

In other preferred embodiments, the cell system is an extract of a host cell that was grown under defined conditions, and the method involves measuring transcription or translation *in vitro.* Such defined conditions are selected so that transcription or translation of the reporter is increased by the addition of a transcription inhibitor or a translation inhibitor to the cell extract.

One such method for identifying antiviral (known and unknown) agents relies upon a transcription-responsive gene product. This method involves constructing a host cell in which the production of a reporter molecule, measured as a percentage of over-all transcription, increases under conditions in which overall host cell transcription is reduced. Specifically, the reporter molecule is encoded by a nucleic acid either transcriptionally linked to a sequence constructed and arranged to cause a relative increase in the production of the reporter molecule when overall transcription is reduced.

Preferably, the overall transcription is measured by the expression of a second indicator gene whose expression, when measured as a percentage of overall transcription, remains constant when the overall transcription is reduced. The method further involves contacting the host cell with a candidate agent, and determining whether the agent increases the production of the first reporter molecule in the host cell.

In a preferred embodiment, the reporter molecule is itself the transcription-responsive gene product whose production increases when overall transcription is reduced. In another preferred embodiment, the reporter is a different molecule whose production is linked to that of the transcription-responsive gene product. Such linkage between the reporter and the transcription-responsive gene product can be achieved in several ways. A gene sequence encoding the reporter may, for example, be fused to part or all of the gene encoding the transcription-responsive gene product and/or to part or all of the genetic elements which control the production of the gene product. Alternatively, the transcription-responsive gene product may stimulate transcription of the gene encoding the reporter, either directly or indirectly.

Alternatively. the method for identifying antiviral (known and unknown) agents relies upon a translation-responsive gene product. This method involves constructing a host cell in which the production of a reporter molecule, measured as a percentage of over-all translation, increases under conditions in which overall host cell translation is reduced. Specifically, the reporter molecule is encoded by nucleic acid either translationally linked or transcriptionally linked to a sequence constructed and arranged to cause a relative increase in the production of the reporter molecule when overall translation is reduced. Preferably, the overall translation is measured by the expression of a second indicator gene whose expression, when measured as a percentage of overall translation, remains constant when the overall translation is reduced. The method further involves contacting the host cell with a candidate agent, and determining whether the agent increases the production of the first reporter molecule in the host cell.

In a preferred embodiment, the reporter molecule is itself the translation-responsive gene product whose production increases when overall translation is reduced. In another preferred embodiment, the reporter is a different molecule whose production is linked to that of the translation-responsive gene product. Such linkage between the reporter and the translation-responsive gene product can be achieved in several ways. A gene sequence encoding the reporter may, for example, be fused to part or all of the gene encoding the translation-responsive gene product and/or to part or all of the genetic elements which control the production of the gene product. Alternatively, the translation-responsive gene product may stimulate translation of the gene encoding the reporter, either directly or indirectly.

Preferably a wide variety of reporters may be used, with preferred reporters providing conveniently detectable signals (eg. by spectroscopy). By way of example, a reporter gene may encode an enzyme which catalyses a reaction which alters light absorption properties.

Examples of reporter molecules include but are not limited to β-galactosidase, invertase, green fluorescent protein, luciferase, chloramphenicol, acetyltransferase, β-glucuronidase, exo-glucanase and glucoamylase. Alternatively, radiolabeled or fluorescent tag-labeled nucleotides can be incorporated into nascent transcripts which are then identified when bound to oligonucleotide probes.

In one preferred embodiment, the production of the reporter molecule is measured by the enzymatic activity of the reporter gene product, such as β-galactosidase.

In another preferred embodiment of the present invention a method of identifying agents that affect the target nucleotide sequence and/or EP comprises (i) arranging cells from a target organism, such as a virus or an individual, in a physical matrix such as a microtiter plate wherein each of the cells contains a recombinant construct comprising a reporter gene operatively linked to a different endogenous transcriptional regulatory element of said target organism such that said transcriptional regulatory element regulates the expression of said reporter gene; (ii) including a sufficient number of different recombinant cells to provide an ensemble of transcriptional regulatory elements of said organism sufficient to model the transcriptional responsiveness of said organism to a candidate agent under one or more of a variety of physical conditions; (iii) contacting a plurality of cells from the target organism with a candidate agent which may provoke a specific cellular response; and (iv) detecting a reporter gene product signal for each cell.

Each cell is contacted with a candidate agent which can provoke a specific cellular response. Preferred agents are pharmaceutical agents, particularly therapeutic agents. The agent may be an organic compound or an inorganic compound. The agent may induce a complex response pattern of induction, repression, silence or which may be expressed as an increase in reporter activity, a decrease in reporter activity or no change at all in reporter activity. The response profile is designed to reflect the cell's transcriptional adjustments to maintain homeostasis in the presence of the agent.

After contacting the cells with the candidate agent, the reporter gene product signals from one or more of the cells is measured to determine a stimulated response profile. The basal of background response profile is then compared with (e.g. subtracted from, or divided into) the stimulated response profile to identify the cellular response profile to the candidate agent. The cellular response may be characterised in a number of ways. For example, the basal profile may be subtracted from the stimulated profile to yield a net stimulation profile. In another embodiment, the stimulated profile may be divided by the basal profile to yield an induction ratio profile. Such comparison profiles provide an estimate of the physiological specificity of the candidate agent.

Response profiles for unknown agents (e.g., new chemicals, unknown compounds or unknown mixtures) may be analysed by comparing the new stimulus response profiles with response profiles to known chemical stimuli.

In another embodiment of the present invention, a selection of hybridisation probes corresponding to a predetermined population of genes of the selected viral organism may be used to specifically detect changes in gene transcription which result from exposing the selected organism or cells thereof to a candidate agent. In this embodiment, one or more cells derived from the organism is exposed to the candidate agent *in vivo* or *ex vivo* under conditions wherein the agent effects a change in gene transcription in the cell to maintain homeostasis. Thereafter, the gene transcripts, primarily mRNA, of the cell or cells are isolated by conventional means. The isolated transcripts or cDNAs complementary thereto are then contacted with an ordered matrix of hybridisation probes, each probe being specific for a different one of the transcripts, under conditions wherein each of the transcripts hybridises with a corresponding one of the probes to form hybridisation pairs. The ordered matrix of probes provides, in aggregate, complements for an ensemble of genes of the organism sufficient to model the transcriptional responsiveness of the organism to a candidate agent. The probes are generally immobilised and arrayed onto a solid substrate such as a microtiter plate. Specific hybridisation may be effected, for example, by washing the hybridised matrix with excess non-specific oligonucleotides. A hybridisation signal is then detected at each hybridisation pair to obtain a transcription signal profile. A wide variety of hybridisation signals may be used. In one preferred embodiment, the cells are pre-labeled with radionucleotides such that the gene transcripts provide a radioactive signal that can be detected in the hybridisation pairs. The transcription signal profile of the agent-treated cells is then compared with a transcription signal profile of negative control cells to obtain a specific transcription response profile to the candidate agent.

The nucleotide sequences of the present invention may be engineered in order to alter the EP coding sequence for a variety of reasons, including but not limited to, alterations which modify the cloning, processing and/or expression of the gene product. For example, mutations may be introduced using techniques which are well known in the art, e.g., site-directed mutagenesis to insert new restriction sites, to alter glycosylation patterns or to change codon preference.

In another embodiment of the invention, an EP natural, modified or recombinant sequence may be ligated to a heterologous sequence to encode a fusion protein. For example, for screening of peptide libraries for inhibitors of the EP activity, it may be useful to encode a chimeric EP expressing a heterologous epitope that is recognized by a commercially available antibody.

The present invention thus provides for fusion proteins comprising the respected target or an enzymatically active fragment or derivative thereof linked to an affinity tag such as glutathione-S-transferase (GST), biotin, His6, *myc* and hemagglutinin (HA) (as described in Wilson *et al* (1984 Cell 37 767). Preferably the fused recombinant protein comprises an antigenic co-protein such as GST, β-galactosidase or the lipoprotein D from *Haemophilus influenzae* which are relatively large co-proteins, which solubilise and facilitate production and purification thereof. Alternatively, the fused protein may comprise a carrier protein such as bovine serum albumin (BSA) or keyhole limpet haemocyanin (KLH). In certain embodiments of the present invention, the marker sequence is a hexa-histidine peptide, as provided in the pQE vector (Qiagen Inc) and described in Gentz *et al* (1989 PNAS 86: 821-824). Such fusion proteins are readily expressable in yeast culture (as described in Mitchell *et al* 1993 Yeast 5: 715-723) and are easily purified by affinity chromatography. A fusion protein may also be engineered to contain a cleavage site located between the nucleotide sequence encoding the EP and the heterologous protein sequence, so that the EP may be cleaved and purified away from the heterologous moiety. In another embodiment, an assay for the target protein may be conducted using the entire, bound fusion protein. Alternatively, the co-protein may act as an adjuvant in the sense of providing a generalised stimulation of the immune system. The co-protein may be attached to either the amino or carboxy terminus of the first protein.

Although the presence/absence of marker gene expression suggests that the nucleotide sequence and/or its EP is also present, its presence and expression should be confirmed. For example, if the EP coding sequence is inserted within a marker gene sequence, recombinant cells containing the EP coding regions may be identified by the absence of marker gene function. Alternatively, a marker gene may be placed in tandem with a the EP coding sequence under the control of a single promoter. Expression of the marker gene in response to induction or selection usually indicates expression of the EP as well.

Additional methods to quantitate the expression of a particular molecule include radiolabeling (Melby PC *et al* 1993 J Immunol Methods 159:235-44) or biotinylating (Duplaa C *et al* 1993 Anal Biochem 229-36) nucleotides, coamplification of a control nucleic acid, and standard curves onto which the experimental results are interpolated. Quantitation of multiple samples may be speeded up by running the assay in an ELISA format where the oligomer of interest is presented in various dilutions and a spectrophotometric or calorimetric response gives rapid quantitation.

The present invention also relates to expression vectors and host cells comprising polynucleotide sequences encoding the EP or variant, homologue, fragment or derivative thereof for the *in vivo* or *in vitro* production of the EP with a view to screening for agents that can affect the EP expression or activity.

In accordance with the present invention, the EP polynucleotide sequences which encode the EP, fragments of the polypeptide, fusion proteins or functional equivalents thereof, may be used to generate recombinant DNA molecules that direct the expression of the EP in appropriate host cells. In one embodiment of the present invention, polynucleotide encoding the EP of the present invention is operably linked to a promoter sequence capable of directing expression of the polynucleotide in a suitable host cell. When inserted into the host cell, the transformed host cell may be cultured under suitable conditions until sufficient levels of the polypeptide are achieved after which the cells may be lysed and the EP isolated. The EP itself may be isolated from host cells using familiar techniques for the fragmentation and sequence analysis of genetic material including the use of oligonucleotide probes and PCR amplification techniques to identify known target sequences or suspected sequences having substantial sequence homology with the target sequence.

Altered EP polynucleotide sequences which may be used in accordance with the invention include deletions, insertions or substitutions of different nucleotide residues resulting in a polynucleotide that encodes the same or a functionally equivalent the EP.

The protein may also have deletions, insertions or substitutions of amino acid residues which produce a silent change and result in a functionally equivalent EP. Deliberate amino acid substitutions may be made on the basis of similarity in polarity, charge, solubility, hydrophobicity, hydrophilicity, and/or the amphipathic nature of the residues as long as the biological activity of the EP is retained. For example, negatively charged amino acids include aspartic acid and glutamic acid; positively charged amino acids include lysine and arginine; and amino acids with uncharged polar head groups having similar hydrophilicity values include leucine, isoleucine, valine, glycine, alanine, asparagine, glutamine, serine, threonine, phenylalanine, and tyrosine.

Due to the inherent degeneracy of the genetic code, other DNA sequences which encode substantially the same or a functionally equivalent amino acid sequence, may be used to clone and express the EP. As will be understood by those of skill in the art, it may be advantageous to produce the EP - encoding nucleotide sequences possessing non-naturally occurring codons. Codons preferred by a particular prokaryotic or eukaryotic host (Murray E *et al* (1989) Nuc Acids Res 17:477-508) can be selected, for example, to increase the rate of the EP expression or to produce recombinant RNA transcripts having desirable properties, such as a longer half-life, than transcripts produced from naturally occurring sequence.

In an alternative embodiment of the invention, the coding sequence of the EP may be synthesized, in whole or in part, using chemical methods well known in the art (see Caruthers MH *et al* (1980) Nuc Acids Res Symp Ser 215-23, Horn T *et al* (1980) Nuc Acids Res Symp Ser 225-232). Alternatively, the EP itself may be produced using chemical methods to synthesize the EP amino acid sequence, in whole or in part. For example, peptides can be synthesized by solid phase techniques, cleaved from the resin, and purified by preparative high performance liquid chromatography (e.g., Creighton (1983) Proteins Structures And Molecular Principles, WH Freeman and Co, New York NY). The composition of the synthetic peptides may be confirmed by amino acid analysis or sequencing (e.g., the Edman degradation procedure; Creighton, *supra).*

Direct peptide synthesis can be performed using various solid-phase techniques (Roberge JY *et al* (1995) Science 269: 202-204) and automated synthesis may be achieved, for example, using the ABI 43 1 A Peptide Synthesizer (Perkin Elmer) in accordance with the instructions provided by the manufacturer. Additionally, the amino acid sequence of the EP, or any part thereof, may be altered during direct synthesis and/or combined using chemical methods with a sequence from other subunits, or any part thereof, to produce a variant polypeptide.

The EP may also be expressed as a recombinant protein with one or more additional polypeptide domains added to facilitate protein purification. Such purification facilitating domains include, but are not limited to, metal chelating peptides such as histidine-tryptophan modules that allow purification on immobilized metals (Porath J (1992) Protein Expr Purif 3 -.26328 1), protein A domains that allow purification on immobilized immunoglobulin, and the domain utilized in the FLAGS extension/affinity purification system (Immunex Corp, Seattle, WA). The inclusion of a cleavable linker sequence such as Factor XA or enterokinase (Invitrogen, San Diego, CA) between the purification domain and the EP is useful to facilitate purification.

Host cells transformed with the EP nucleotide coding sequence may be cultured under conditions suitable for the expression and recovery of the encoded protein from cell culture. The protein produced by a recombinant cell may be secreted or may be contained intracellularly depending on the sequence and/or the vector used. As will be understood by those of skill in the art, expression vectors containing the EP coding sequences can be designed with signal sequences which direct secretion of the EP coding sequences through a particular prokaryotic or eukaryotic cell membrane. Other recombinant constructions may join the EP coding sequence to nucleotide sequence encoding a polypeptide domain which will facilitate purification of soluble proteins (Kroll *DJ et al* (1993) DNA Cell Biol 12:441-53, see also above discussion of vectors containing fusion proteins).

A variety of protocols for detecting and measuring the expression of the EP polypeptide, using either polyclonal or monoclonal antibodies specific for the protein are known in the art. Examples include enzyme-linked immunosorbent assay (ELISA), radioimmunoassay (RIA) and fluorescent activated cell sorting (FACS). A two-site, monoclonal-based immunoassay utilizing monoclonal antibodies reactive to two non-interfering epitopes on the EP polypeptides is preferred, but a competitive binding assay may be employed. These and other assays are described, among other places, in Hampton R *et al* (1990, Serological Methods, A Laboratory Manual. APS Press, St Paul MN) and Maddox DE *et al* (1983, J Exp Med 15 8:121 1).

In an embodiment of the present invention, the EP or a variant, homologue, fragment or derivative thereof and/or a cell line that expresses the EP or variant, homologue, fragment or derivative thereof may be used to screen for antibodies, peptides, or other agent, such as organic or inorganic molecules, that act as modulators of the EP activity, thereby identifying a therapeutic agent capable of modulating the levels of the EP. For example, anti-EP antibodies capable of neutralizing the activity of the EP may be used to inhibit the EP activity thereby increasing their levels. Alternatively, screening of peptide libraries or organic libraries made by combinatorial chemistry with recombinantly expressed EP or a variant, homologue, fragment or derivative thereof or cell lines expressing the EP or a variant, homologue, fragment or derivative thereof may be useful for identification of therapeutic agents that function by modulating the EP activity. Synthetic compounds, natural products, and other sources of potentially biologically active materials can be screened in a number of ways deemed to be routine to those of skill in the art. For example, nucleotide sequences encoding the N-terminal region of the EP may be expressed in a cell line which can be used for screening of allosteric modulators, either agonists or antagonists, of the EP activity. Alternatively, nucleotide sequences encoding the conserved catalytic domain of the EP can be expressed in cell lines and used to screen for inhibitors of the EP activity.

Procedures well known in the art may be used for the production of antibodies to the EP polypeptides. Such antibodies include, but are not limited to, polyclonal, monoclonal, chimeric, single chain, Fab fragments and fragments produced by a Fab expression library. Neutralizing antibodies, i.e., those which inhibit biological activity of the EP polypeptides, are especially preferred for diagnostics and therapeutics.

For the production of antibodies, various hosts including goats, rabbits, rats, mice, etc. may be immunized by injection with the EP polypeptide or any portion, variant, homologue, fragment or derivative thereof or oligopeptide which retains immunogenic properties. Depending on the host species, various adjuvants may be used to increase immunological response. Such adjuvants include, but are not limited to, Freund's, mineral gels such as aluminium hydroxide, and surface active substances such as lysolecithin, pluronic polyols, polyanions, peptides, oil emulsions, keyhole limpet hemocyanin, and dinitrophenol. BCG *(Bacilli Calmette-Guerin)* and *Corynebacterium parvum* are potentially useful human adjuvants which may be employed if purified the EP polypeptide is administered to immunologically compromised individuals for the purpose of stimulating systemic defence.

Monoclonal antibodies to the EP polypeptide may be prepared using any technique which provides for the production of antibody molecules by continuous cell lines in culture. These include, but are not limited to, the hybridoma technique originally described by Koehler and Milstein (1975 Nature 256:495-497), the human B-cell hybridoma technique (Kosbor *et al* (1983) Immunol Today 4:72; Cote *et al* (1983) Proc Natl Acad Sci 80:2026-2030) and the EBV-hybridoma technique (Cole *et al* (1985) Monoclonal Antibodies and Cancer Therapy, Alan R Liss Inc, pp 77-96). In addition, techniques developed for the production of "chimeric antibodies", the splicing of mouse antibody genes to human antibody genes to obtain a molecule with appropriate antigen specificity and biological activity can be used (Morrison *et al* (1984) Proc Natl Acad Sci 81:6851-6855; Neuberger *et al* (1984) Nature 312:604-608; Takeda *et al* (1985) Nature 314:452-454). Alternatively, techniques described for the production of single chain antibodies (US Patent No. 4,946,779) can be adapted to produce the EP specific single chain antibodies.

Antibodies may also be produced by inducing *in vivo* production in the lymphocyte population or by screening recombinant immunoglobulin libraries or panels of highly specific binding reagents as disclosed in Orlandi *et al* (1989, Proc Natl Acad Sci 86: 3833-3837), and Winter G and Milstein C (1991; Nature 349:293-299).

Antibody fragments which contain specific binding sites for the EP may also be generated. For example, such fragments include, but are not limited to, the F(ab')₂ fragments which can be produced by pepsin digestion of the antibody molecule and the Fab fragments which can be generated by reducing the disulfide bridges of the F(ab')₂ fragments. Alternatively, Fab expression libraries may be constructed to allow rapid and easy identification of monoclonal Fab fragments with the desired specificity (Huse WD *et al* (1989) Science 256:1275-128 1).

The EP-specific antibodies are useful for the diagnosis of conditions and diseases associated with expression of the EP polypeptide. A variety of protocols for competitive binding or immunoradiometric assays using either polyclonal or monoclonal antibodies with established specificities are well known in the art. Such immunoassays typically involve the formation of complexes between the EP polypeptides and its specific antibody (or similar the EP-binding molecule) and the measurement of complex formation. A two-site, monoclonal based immunoassay utilizing monoclonal antibodies reactive to two noninterfering epitopes on a specific EP protein is preferred, but a competitive binding assay may also be employed. These assays are described in Maddox DE *et al* (1983, J Exp Med 158:121 1).

Anti-EP antibodies are useful for the diagnosis of inflammation, conditions associated with proliferation of hematopoietic cells and HIV infection or other disorders or diseases characterized by abnormal expression of the EP. Diagnostic assays for an EP include methods utilizing the antibody and a label to detect an EP polypeptide in human body fluids, cells, tissues or sections or extracts of such tissues. The polypeptides and antibodies of the present invention may be used with or without modification. Frequently, the polypeptides and antibodies will be labeled by joining them, either covalently or noncovalently, with a reporter molecule. As already outlined, a wide variety of reporter molecules are known to those of skill in the art.

A variety of protocols for measuring an EP polypeptide, using either polyclonal or monoclonal antibodies specific for the respective protein are known in the art. Examples include enzyme-linked immunosorbent assay (ELISA), radioimmunoassay (RIA), competitive binding assays, Western Blot analysis and fluorescent activated cell sorting (FACS). A two-site, monoclonal-based immunoassay utilizing monoclonal antibodies reactive to two non-interfering epitopes on an the EP polypeptide is preferred, but a competitive binding assay may be employed. These assays are described, among other places, in Maddox, DE *et al* (1983, J Exp Med 15 8:121 1).

Additionally, the present invention relates to the use of an EP polypeptide, or variant, homologue, fragment or derivative thereof, to produce anti-EP antibodies which can, for example, be used diagnostically to detect and quantitate the EP levels in disease states.

Another aspect of the subject invention is to provide for nucleic acid hybridization or PCR probes which are capable of detecting polynucleotide sequences, including genomic sequences, encoding the EP coding region or closely related molecules, such as alleles. The specificity of the probe, i.e., whether it is derived from a highly conserved, conserved or non-conserved region or domain, and the stringency of the hybridization or amplification (high, intermediate or low) will determine whether the probe identifies only naturally occurring EP coding sequence, or related sequences. Probes for the detection of related nucleic acid sequences are selected from conserved or highly conserved nucleotide regions of the EP family members, such as the 3' region, and such probes may be used in a pool of degenerate probes. For the detection of identical nucleic acid sequences, or where maximum specificity is desired, nucleic acid probes are selected from the non-conserved nucleotide regions or unique regions of the EP polynucleotides. As used herein, the term "non-conserved nucleotide region" refers to a nucleotide region that is unique to the EP coding sequence disclosed herein and does not occur in related family members.

An EP nucleic acid antisense molecule may be used to modify the expression of or block the activity of the EP in conditions where it would be deemed preferable. Alternatively, these sequences can be used as a probe, or for amplifying all or part of such sequence when used as a polymerase chain reaction primer. An EP antisense molecule may also provide the basis for treatment of various abnormal conditions related to, for example, increased the EP activity - such as found in mycoses, viral infection or infestations.

A wide variety of labels and conjugation techniques are known by those skilled in the art and can be used in various nucleic and amino acid assays. Means for producing labelled hybridization or PCR probes for detecting the EP polynucleotide sequences include oligolabelling, nick translation, end-labelling or PCR amplification using a labelled nucleotide. Alternatively, the EP coding sequence, or any portion of it, may be cloned into a vector for the production of an mRNA probe. Such vectors are known in the art, are commercially available, and may be used to synthesize RNA probes *in vitro* by addition of an appropriate RNA polymerase such as T7, T3 or SP6 and labeled nucleotides.

A number of companies such as Pharmacia Biotech (Piscataway, NJ), Promega (Madison, WI), and US Biochemical Corp (Cleveland, OH) supply commercial kits and protocols for these procedures. Suitable reporter molecules or labels include those radionuclides, enzymes, fluorescent, chemiluminescent, or chromogenic agents as well as substrates, cofactors, inhibitors, magnetic particles and the like. Patents teaching the use of such labels include US-A-3,817,837; US-A-3,850,752; US-A-3,939,350; US-A-3,996,345; US-A-4,277,437; US-A-4,275,149 and US-A-4,366,241. Also, recombinant immunoglobulins may be produced as shown in US-A-4,816,567.

PCR as described in US-A-4,683,195; US-A-4,800,195; and US-A-4,965,188 provides additional uses for oligonucleotides based upon the EP sequence. Such oligomers are generally chemically synthesized, but they may be generated enzymatically or produced from a recombinant source. Oligomers generally comprise two nucleotide sequences, one with sense orientation (5'->3') and one with antisense (3'<-5') employed under optimized conditions for identification of a specific gene or condition. The same two oligomers, nested sets of oligomers, or even a degenerate pool of oligomcrs may be employed under less stringent conditions for detection and/or quantitation of closely related DNA or RNA sequences.

The nucleic acid sequence for the EP can also be used to generate hybridization probes as previously described, for mapping the endogenous genomic sequence. The sequence may be mapped to a particular chromosome or to a specific region of the chromosome using well known techniques. These include *in situ* hybridization to chromosomal spreads (Verma *et al* (1988) Human Chromosomes: A Manual of Basic Techniques, Pergamon Press, New York City), flow-sorted chromosomal preparations, or artificial chromosome constructions such as YACs, bacterial artificial chromosomes (BACs), bacterial PI constructions or single chromosome cDNA libraries.

*In situ* hybridization of chromosomal preparations and physical mapping techniques such as linkage analysis using established chromosomal markers are invaluable in extending genetic maps. Examples of genetic maps can be found in Science (1995; 270:410f and 1994; 265:1981f). Often the placement of a gene on the chromosome of another mammalian species may reveal associated markers even if the number or arm of a particular human chromosome is not known. New sequences can be assigned to chromosomal arms, or parts thereof, by physical mapping. This provides valuable information to investigators searching for disease genes using positional cloning or other gene discovery techniques. Once a disease or syndrome, such as ataxia telangiectasia (AT), has been crudely localized by genetic linkage to a particular genomic region, for example, AT to 1Iq22-23 (Gatti *et al* (1988) Nature 336:577-580), any sequences mapping to that area may represent associated or regulatory genes for further investigation. The nucleotide sequence of the subject invention may also be used to detect differences in the chromosomal location due to translocation, inversion, etc between normal, carrier or affected individuals.

An abnormal level of nucleotide sequences encoding an EP in a biological sample may reflect a chromosomal aberration, such as a nucleic acid deletion or mutation. Accordingly, nucleotide sequences encoding an EP provide the basis for probes which can be used diagnostically to detect chromosomal aberrations such as deletions, mutations or chromosomal translocations in the gene encoding the EP. The EP gene expression may be altered in such disease states or there may be a chromosomal aberration present in the region of the gene encoding an the EP.

Such assays may be tailored to evaluate the efficacy of a particular therapeutic treatment regime and may be used in animal studies, in clinical trials, or in monitoring the treatment of an individual. In order to provide a basis for the diagnosis of disease, a normal or standard profile for the EP must be established. This is accomplished by combining body fluids or cell extracts taken from normal subjects, either animal or human, with the EP or a portion thereof, under conditions suitable for hybridization or amplification. Standard hybridization may be quantified by comparing the values obtained for normal subjects with a dilution series of positive controls run in the same experiment where a known amount of purified EP is used. Standard values obtained from normal samples may be compared with values obtained from samples from subjects potentially affected by a disorder or disease related to expression of the EP coding sequence. Deviation between standard and subject values establishes the presence of the disease state. If disease is established, an existing therapeutic agent is administered, and treatment profile or values may be generated. Finally, the assay may be repeated on a regular basis to evaluate whether the values progress toward or return to the normal or standard pattern. Successive treatment profiles may be used to show the efficacy of treatment over a period of several days or several months.

An EP encoding polynucleotide sequence may be used for the diagnosis of diseases resulting from expression of the EP. For example, polynucleotide sequences encoding the EP may be used in hybridization or PCR assays of tissues from biopsies or autopsies or biological fluids, such as serum, synovial fluid or tumor biopsy, to detect abnormalities in the expression of the EP. The form of such qualitative or quantitative methods may include Southern or Northern analysis, dot blot or other membrane-based technologies; PCR technologies; dip stick, pin or chip technologies; and ELISA or other multiple sample formal technologies. All of these techniques are well known in the art and are in fact the basis of many commercially available diagnostic kits.

Included in the scope of the invention are oligonucleotide sequences, antisense RNA and DNA molecules and ribozymes, which function to destabilize the EP mRNA or inhibit translation of the EP. Such nucleotide sequences may be used in conditions where it would be preferable to increase the EP levels.

Expression vectors derived from retroviruses, adenovirus, herpes or vaccinia viruses, or from various bacterial plasmids, may be used for delivery of recombinant the EP sense or antisense molecules to the targeted cell population. Methods which are well known to those skilled in the art can be used to construct recombinant vectors containing the EP. Alternatively, recombinant EP can be delivered to target cells in liposomes.

The full length cDNA sequence and/or its regulatory elements enable researchers to use the EP as a tool in sense (Youssoufian H and HF Lodish 1993 Mol Cell Biol 13:98-104) or antisense (Eguchi *et al* (1991) Ann Rev Biochem 60:631-652) investigations of gene function. Oligonucleotides, designed from the cDNA or control sequences obtained from the genomic DNA can be used *in vitro* or *in vivo* to inhibit expression. Such technology is now well known in the art, and sense or antisense oligonucleotides or larger fragments can be designed from various locations along the coding or control regions.

Additionally, the EP can be modulated by transfecting a cell or tissue with expression vectors which express high levels of an EP fragment in conditions where it would be preferable to block the EP activity. Such constructs can flood cells with untranslatable sense or antisense sequences. Even in the absence of integration into the DNA, such vectors may continue to transcribe RNA molecules until all copies of the vector are disabled by endogenous nucleases. Such transient expression may last for a month or more with a non-replicating vector and even longer if appropriate replication elements are part of the vector system.

Modifications of gene expression can be obtained by designing antisense sequences to the control regions of the EP gene, such as the promoters, enhancers, and introns.

Oligonucleotides derived from the transcription initiation site, e.g., between -10 and +10 regions of the leader sequence, are preferred. Antisense RNA and DNA molecules may also be designed to block translation of mRNA by preventing the transcript from binding to ribosomes. Similarly, inhibition can be achieved using Hogeboom base-pairing methodology, also known as "triple helix" base pairing. Triple helix pairing compromises the ability of the double helix to open sufficiently for the binding of polymerases, transcription factors, or regulatory molecules.

Included in the scope of the invention are oligonucleotide sequences, antisense RNA and DNA molecules and ribozymes, which function to destabilize the EP mRNA or inhibit translation of the EP. Such nucleotide sequences may be used in conditions where it would be preferable to increase the EP levels.

Ribozymes are enzymatic RNA molecules capable of catalyzing the specific cleavage of RNA. The mechanism of ribozyme action involves sequence specific hybridization of the ribozyme molecule to complementary target RNA, followed by a endonucleolytic cleavage. Within the scope of the invention are engineered hammerhead motif ribozyme molecules that specifically and efficiently catalyze endonucleolytic cleavage of the EP RNA sequences.

Specific ribozyme cleavage sites within any potential RNA target are initially identified by scanning the target molecule for ribozyme cleavage sites which include the following sequences, GUA, GUU and GUC. Once identified, short RNA sequences of between 15 and 20 ribonucleotides corresponding to the region of the target gene containing the cleavage site may be evaluated for secondary structural features which may render the oligonucleotide sequence inoperable. The suitability of candidate targets may also be evaluated by testing accessibility to hybridization with complementary oligonucleotides using ribonuclease protection assays.

Both antisense RNA and DNA molecules and ribozymes of the invention may be prepared by any method known in the art for the synthesis of RNA molecules. These include techniques for chemically synthesizing oligonucleotides such as solid phase phosphoramidite chemical synthesis. Alternatively, RNA molecules may be generated by *in vitro* or *in vivo* transcription of DNA sequences encoding the antisense RNA molecule. Such DNA sequences may be incorporated into a wide variety of vectors with suitable RNA polymerase promoters such as T7 or SP6. Alternatively, antisense cDNA constructs that synthesize antisense RNA constitutively or inducibly can be introduced into cell lines, cells or tissues.

DNA molecules may be modified to increase intracellular stability and half-life. Possible modifications include, but are not limited to, the addition of flanking sequences of the 5' and/or 3' ends of the molecule or the use of phosphorothioate or 2' O-methyl rather than phosphodiesterase linkages within the backbone of the molecule.

Methods for introducing vectors into cells or tissue include those methods discussed herein. In addition, several of these transformation or transfection methods are equally suitable for *ex vivo* therapy,

The present invention also relates to the use of genetically engineered host cells expressing an EP or variant, homologue, fragment or derivative thereof in screening methods for the identification of inhibitors and antagonists of the EP that would modulate the EP activity. Such genetically engineered host cells could be used to screen peptide libraries or organic molecules capable of modulating the EP activity. Antagonists and inhibitors of the EP, such as antibodies, peptides or small organic molecules will provide the basis for pharmaceutical compositions for the treatment of diseases associated with, for example, mycoses, viral infections and infestation. Such inhibitors or antagonists can be administered alone or in combination with other therapeutics for the treatment of such diseases.

Alternatively, host cells which contain the coding sequence for the EP and express the EP coding regions may be identified by a variety of procedures known to those of skill in the art. These procedures include, but are not limited to, DNA-DNA or DNA-RNA hybridization and protein bioassay or immunoassay techniques which include membrane-based, solution-based, or chip-based technologies for the detection and/or quantification of the nucleic acid or protein.

An EP polypeptide, its immunogenic fragments or oligopeptides thereof can be used for screening therapeutic compounds in any of a variety of drug screening techniques. The polypeptide employed in such a test may be free in solution, affixed to a solid support, borne on a cell surface, or located intracellularly. The abolition of activity or the formation of binding complexes between the EP polypeptide and the agent being tested may be measured.

In order to provide a basis for the diagnosis of disease, normal or standard values from an EP polypeptide expression must be established. This is accomplished by combining body fluids or cell extracts taken from normal subjects, either animal or human, with antibody to an EP polypeptide under conditions suitable for complex formation which are well known in the art. The amount of standard complex formation may be quantified by comparing it to a dilution series of positive controls where a known amount of antibody is combined with known concentrations of a purified EP polypeptide. Then, standard values obtained from normal samples may be compared with values obtained from samples from subjects potentially affected by a disorder or disease related to an EP polypeptide expression. Deviation between standard and subject values establishes the presence of the disease state.

Alternatively, phage display can be employed in the identification of candidate agents which are anti-viral inhibitory molecules. Phage display is a protocol of molecular screening which utilises recombinant bacteriophage. The technology involves transforming bacteriophage with a gene that encodes an appropriate ligand (in this case a candidate agent) capable of binding to the EP (or a variant, homologue, fragment or derivative therof) or the nucleotide sequence (or a variant, homologue, fragment or derivative therof) encoding same. The transformed bacteriophage (which preferably is tethered to a solid support) expresses the appropriate ligand (such as the candidate agent) and displays it on their phage coat. The entity or entities (such as cells) bearing the target molecules which recognises the candidate agent are isolated and amplified. The successful candidate agents are then characterised. Phage display has advantages over standard affinity ligand screening technologies. The phage surface displays the candidate agent in a three dimensional configuration, more closely resembling its naturally occuring conformation. This allows for more specific and higher affinity binding for screening purposes.

Accordingly, the present invention provides a method for screening a plurality of agents for specific binding affinity with the EP, or a portion, variant, homologue, fragment or derivative thereof, comprising providing a plurality of agents; combining the EP or a portion, variant, homologue, fragment or derivative thereof with each of a plurality of agents for a time sufficient to allow binding under suitable conditions; and detecting binding of the EP, or portion, variant, homologue, fragment or derivative thereof, to each of the plurality of agents, thereby identifying the agent or agents which specifically bind the EP. In such an assay, the plurality of agents may be produced by combinatorial chemistry techniques known to those of skill in the art.

Another technique for screening provides for high throughput screening of agents having suitable binding affinity to the EP polypeptides and is based upon the method described in detail in Geysen, European Patent Application 84/03564, published on September 13, 1984. In summary, large numbers of different small peptide test compounds are synthesized on a solid substrate, such as plastic pins or some other surface. The peptide test agents are reacted with the EP fragments and washed. A bound EP is then detected - such as by appropriately adapting methods well known in the art. A purified EP can also be coated directly onto plates for use in the aforementioned drug screening techniques. Alternatively, non-neutralizing antibodies can be used to capture the peptide and immobilize it on a solid support.

This invention also contemplates the use of competitive drug screening assays in which neutralizing antibodies capable of binding the EP specifically compete with a test compound for binding the EP. In this manner, the antibodies can be used to detect the presence of any peptide which shares one or more antigenic determinants with an the EP.

An EP polynucleotide, or any part thereof, may provide the basis for a diagnostic and/or a therapeutic compound. For diagnostic purposes, the EP polynucleotide sequences may be used to detect and quantitate gene expression in conditions, disorders or diseases in which the EP activity may be implicated, for example, in viral infection or infestations.

The present invention also provides a diagnostic composition for the detection of the EP polynucleotide sequences. The diagnostic composition may comprise the polynucleotide SEQ ID No. 1 and/or SEQ ID No.3, or a variant, homologue, fragment or derivative thereof, or a sequence capable of hybridising to all or part of SEQ ID No. 1 and/or SEQ ID No.3, or an allelic variation thereof.

The putative agent to be screened may be any suitable candidiate chemical entity - such as a small organic molecule, small inorganic molecule, a large organic molecule, a large in organic molecule etc. Examples of putative agents may include nucleotide sequences - which may be sense or anti-sense sequences, anti-bodies, enzymes, proteins, peptides etc.

It is believed that an agent which binds to the nucleotide sequence of the present invention and/or the protein sequence of the present invention and, as a result of which, causes directly or indirectly an adverse effect on the viral infection is potentially useful as an anti-viral agent. If necessary, such agents can be further screened to ensure that they are specific to virally infected cells and have no effect non-infected cells. Thus, the agents could be used as a therapeutic or as a prophylactic. Even if the agents have a detrimental effect on the host cells then they may nevertheless still be useful as a therapeutic - particularly when treating life threatening infections, such as HIV-1 infection.

The present invention also provides a pharmaceutical composition for treating an individual in need of same due to the EP activity comprising administering a therapeutically effective amount of an agent that affects (such as inhibits) said activity and a pharmaceutically acceptable carrier, diluent, excipient or adjuvant.

Acceptable carriers or diluents for therapeutic use are well known in the pharmaceutical art, and are described, for example, in Remington's Pharmaceutical Sciences, Mack Publishing Co. (A. R. Gennaro edit. 1985). Preservatives, stabilizers, dyes and even flavoring agents may be provided in the pharmaceutical composition. Examples of preservatives include sodium benzoate, sorbic acid and esters of p-hydroxybenzoic acid. Antioxidants and suspending agents may be also used.

The pharmaceutical compositions may be for human or animal usage and will typically comprise any one or more of a pharmaceutically acceptable diluent, carrier, excipient or adjuvant. The choice of pharmaceutical carrier, excipient or diluent can be selected with regard to the intended route of administration and standard pharmaceutical practice. The pharmaceutical compositions may comprise as - or in addition to - the carrier, excipient or diluent any suitable binder(s), lubricant(s), suspending agent(s), coating agent(s), solubilising agent(s).

In some embodiments of the present invention, the pharmaceutical compositions will comprise one or more of: an agent that has been screened by an assay of the present invention; an agent that is capable of interacting with any one or more of SEQ ID No. 1, SEQ ID NO. 2, SEQ ID No.3, SEQ ID No.4, including derivatives, fragments, homologues or variants thereof or sequences capable of hybridising to SEQ ID No. 1 and/or SEQ ID No.3 other nucleotide sequences coding for SEQ ID No. 2 and/or SEQ ID No.4.

The present invention also provides the use of an agent to selectively affect the EP activity (such as to inhibit, modulate or agonise) in an individual.

The present invention also provides a method of treating an individual in need of same due to the EP activity comprising administering to said individual an effective amount of the pharmaceutical composition of the present invention.

Typically, a physician will determine the actual dosage which will be most suitable for an individual subject and it will vary with the age, weight and response of the particular patient. The above dosages are exemplary of the average case. There can, of course, be individual instances where higher or lower dosage ranges are merited.

Where appropriate, the pharmaceutical compositions can be administered by inhalation, in the form of a suppository or pessary, topically in the form of a lotion, solution, cream, ointment or dusting powder, by use of a skin patch, orally in the form of tablets containing excipients such as starch or lactose, or in capsules or ovules either alone or in admixture with excipients, or in the form of elixirs, solutions or suspensions containing flavouring or colouring agents, or they can be injected parenterally, for example intracavernosally, intravenously, intramuscularly or subcutaneously. For parenteral administration, the compositions may be best used in the form of a sterile aqueous solution which may contain other substances, for example enough salts or monosaccharides to make the solution isotonic with blood. For buccal or sublingual administration the compositions may be administered in the form of tablets or lozenges which can be formulated in a conventional manner.

The present invention also relates to pharmaceutical compositions comprising effective amounts of inhibitors or antagonists of the EP protein (including anti-sense nucleic acid sequences) in admixture with a pharmaceutically acceptable diluent, carrier, excipient or adjuvant (including combinations thereof).

The present invention relates to pharmaceutical compositions which may comprise all or portions of the EP polynucleotide sequences, the EP antisense molecules, the EP polypeptides, protein, peptide or organic modulators of the EP bioactivity, such as inhibitors, antagonists (including antibodies) or agonists, alone or in combination with at least one other agent, such as a stabilizing compound, and may be administered in any sterile, biocompatible pharmaceutical carrier, including, but not limited to, saline, buffered saline, dextrose, and water.

The present invention further provides diagnostic assays and kits for the detection of the EP in cells and tissues comprising a purified EP which may be used as a positive control, and anti-EP antibodies. Such antibodies may be used in solution-based, membrane-based, or tissue-based technologies to detect any disease state or condition related to the expression of the EP protein or expression of deletions or a variant, homologue, fragment or derivative thereof.

Even though US-A-5738985 does not disclose or suggest the present invention it does provide some background teachings on assays. Some of those teachings have been presented in some of the following description - but with reference to the assay of the present invention and some of the other aspects of the present invention.

Methods to screen potential agents for their ability to disrupt or moderate viral effects on translation can be designed without detailed knowledge of the precise interaction between viral and cellular components, although such a knowledge can certainly be helpful. In principle, many of the numerous methods which have so far been described to identify viral and cellular components involved in effects on translation can be readily adapted to detect interference with the interaction between these components. Thus, for example, if it has been found that viral infection leads to the phosphorylation, dephosphorylation or other modification of a given component, or to a change in its catalytic activity such as the inhibition of that activity, or to enhanced synthesis or degradation of this component, or to any other observable effect described in the foregoing disclosure, them agents can be screened for their ability to prevent or moderate this effect on the component in question. The screening can be performed by adding the test agent to intact cells which have been infected by virus and then examining the component of interest by whatever procedure has been established to demonstrate the viral effect on this component. Alternatively, the screening can be performed by adding the test agent to *in vitro* translation reactions and then proceeding with the established analysis. As another alternative, purified or partially purified components which have been determined to interact with one another by the methods described above can be placed under conditions in which the interaction between them would normally occur, with and without the addition of the test agent, and the procedures previously established to analyze the interaction can be used to assess the impact of the test agent. In this approach, the purified or partially purified components may be prepared by fractionation of extracts from uninfected and infected cells, or they may be obtained by expression of cloned genes or cDNAs or fragments thereof, optionally followed by purification of the expressed material.

Within the broad category of in vitro selection methods, several types of method are likely to be particularly convenient and/or useful for screening test agents. These include but are not limited to methods which measure a binding interaction between two or more components, methods which measure the activity of an enzyme which is one of the interacting components, and methods which measure the activity or expression of "reporter" protein, that is, an enzyme or other detectable or selectable protein, which has been placed under the control of one of the components.

Binding interactions between two or more components can be measured in a variety of ways. One approach is to label one of the components with an easily detectable label, place it together with the other component(s) in conditions under which they would normally interact, perform a separation step which separates bound labeled component from unbound labeled component, and then measure the amount of bound component. The effect of a test agent included in the binding reaction can be determined by comparing the amount of labeled component which binds in the presence of this agent to the amount which binds in its absence.

The separation step in this type of procedure can be accomplished in various ways. In one approach, (one of) the binding partner(s) for the labeled component can be immobilized on a solid phase prior to the binding reaction, and unbound labeled component can be removed after the binding reaction by washing the solid phase. Attachment of the binding partner to the solid phase can be accomplished in various ways known to those skilled in the art, including but not limited to chemical cross-linking, non-specific adhesion to a plastic surface, interaction with an antibody attached to the solid phase, interaction between a ligand attached to the binding partner (such as biotin) and a ligand-binding protein (such as avidin or streptavidin) attached to the solid phase, and so on.

Alternatively, the separation step can be accomplished after the labeled component had been allowed to interact with its binding partner(s) in solution. If the size differences between the labeled component and its binding partner(s) permit such a separation, the separation can be achieved by passing the products of the binding reaction through an ultrafilter whose pores allow passage of unbound labeled component but not of its binding partner(s) or of labeled component bound to its partner(s). Separation can also be achieved using any reagent capable of capturing a binding partner of the labeled component from solution, such as an antibody against the binding partner, a ligand-binding protein which can interact with a ligand previously attached to the binding partner, and so on.

Test methods which rely on measurements of enzyme activity are performed in accordance with the characteristics of the enzyme in each case. As noted above, a variety of enzyme activities can be determined to be involved in the translational effect of a virus, including but not limited to kinases, phosphatases, glycosylases, deglycosylases, transferases, lipases, deoxyribonucleases, ribonucleases, proteases, synthetases, polymerases, and the like, as well as those other enzyme activities noted above. In general, measurements of enzyme activity require the ability to measure the product of the reaction in the presence of other materials, and often to distinguish or separate the product of the reaction from the substrate for the reaction. Methods which may be used to measure reaction products include but are not limited to measurement of the transfer or incorporation of a radioactive or other labeled atom or group, spectrophotometric or colorimetric measurement of the concentration of the product, measurement of light output from a luminescent or chemiluminescent reaction, measurement of fluorescence from a fluorescent product, immunoassays, other immunochemical procedures, and other competitive binding assays. Enzyme activity can also be measured using any of the procedures just mentioned to detect the product of a secondary reaction or reactions which rely on the product of the reaction of interest as a substrate or a cofactor.

In many cases the product of an enzyme reaction can be detected without separating that product from other constituents of the reaction mixture, as for example when an uncolored chromogenic substrate gives rise to a colored product or the absorption spectrum for the product is different from that of the substrate, allowing selection of a wavelength for absorbance measurements of just the product. Immunoassays, other immunochemical procedures and other competitive binding assays can also often be performed without first separating the product of interest.

In other cases it may be necessary to include a separation step or steps to separate the product from other constituents of the reaction mixture before measuring it. In such cases, the necessary separation can be accomplished by a variety of procedures, including but not limited to centrifugation, trichloroacetic acid precipitation, ethanol precipitation, ammonium sulfate precipitation, other differential precipitations, gel filtration, ion exchange chromatography, hydrophobic interaction chromatography, reverse phase chromatography, affinity chromatography, differential extractions, isoelectric focusing, electrophoresis, isotachophoresis, and the like.

In addition to methods which measure the activity of an enzyme implicated in a viral effect on translation, test methods may also be employed which have been configured such that the component(s) implicated in the viral effect controls the activity or expression of a "reporter" protein, that is, an enzyme or other detectable or selectable protein. In the case, for example, where a kinase has been implicated in the viral effect, the test method might be configured in such a way that phosphorylation of a particular protein by the kinase leads to the activation or inhibition of that protein or of some other protein controlled by that protein. In yeast, for example, phosphorylation of eIF2-α by the GCN2 protein (or by mammalian p68 kinase substituting for GCN2) leads to an inhibition of the initiation of translation, which in turn leads to an increase in the synthesis of the GCN4 protein, which in turn induces the synthesis of further proteins involved in amino acid biosynthesis. "Reporter" proteins can be readily fused to the GCN4 protein at the genetic level so that the synthesis of these reporters is effectively induced by the initial phosphorylation event catalyzed by GCN2 or mammalian p68.

Similar approaches can be used to detect modulation by test agents of the activity of a variety of other components which might be implicated in viral effects on translation. The effect of a test agent on a protease, for example, can be monitored by following the survival in an *in vitro* reaction of a reporter protein which is a target for that protease. Similarly, the effect of a test agent on a nuclease can be monitored by following the appearance in an *in vitro* translation reaction or in vitro transcription-translation reaction of a reporter protein translated from a suitably configured coding sequence provided to the reaction.

Proteins suitable for use as reporters in such assays include, but are not limited to, easily assayed enzymes such as β-galactosidase, luciferase, β-glucuronidase, chloramphenicol acetyl transferase, and secreted embryonic alkaline phosphatase; proteins for which immunoassays are readily available such as hormones and cytokines; proteins which confer a selective growth advantage on cells such as adenosine deaminase, aminoglycoside phosphotransferase (the product of the neo gene), dihydrofolate reductase, hygromycin-B-phosphotransferase, thymidine kinase (when used with HAT medium), xanthine-guanine phosphoribosyltransferase (XGPRT), and proteins which provide a biosynthetic capability missing from an auxotroph; proteins which confer a growth disadvantage on cells, for example enzymes that convert non-toxic substrates to toxic products such as thymidine kinase (when used with medium containing bromodeoxyuridine) and orotidine-5'-phosphate decarboxylase (when used with 5-fluoroorotic acid); and proteins which are toxic such as ricin, cholera toxin or diphtheria toxin.

Many of the methods so far described for selecting test agents have involved examining the impact of these agents on the interaction between two or more components in in vitro reactions. The interacting components can also be brought into contact with one another within cells rather than in *in vitro* reactions. In this approach, coding sequence(s) encoding part or all of a component or components would be introduced into a selected type of cell. Coding sequences for this approach include cloned genes or cDNAs or fragments of either or fragments amplified by the polymerase chain reaction or natural RNAs or transcribed RNAs or the like. Several variations of the approach are possible. In one variation, a coding sequence is introduced for a first component into a cell known to contain components with which this; first component will interact. Thus, for example, a coding sequence for a viral component is introduced into a cell which is a normal target for infection by the virus in question. Agents are tested to select those which block the effect of the viral component within the cell into which the coding sequence has been introduced. In another variation, coding sequences for two or more components which interact with one another might be introduced into a cell, and agents tested for their ability to moderate the interaction between these components, this interaction being followed by the procedures previously established as suitable for the purpose. The cell into which the coding sequences are introduced can be one which would normally be a target for infection by the virus in question. Alternatively and usefully, the cell can be one which is easier to grow, manipulate and test such as a yeast cell. Indeed, there are distinct advantages to reconstructing a translation control mechanism in heterologous cells, in which the interactions between the components involved are easier to study than they are when those components are in their normal environment. In the case of yeast, in particular, the powerful genetic approaches available often make it possible to identify and isolate the yeast homologues of genes from higher eukaryotes more quickly than the corresponding genes can be identified in the higher eukaryotes.

From the foregoing it should be apparent that one skilled in the art is able to choose from a wide variety of methods at each stage in the identification of components involved in viral effects on translation, in the characterization of the interaction between these components, and in the implementation of screening tests to select compounds which moderate or abolish the interaction between these components.

In summation, the present invention relates to assay methods for screening for agents useful for treatment of viral infection. The present invention also relates to agents - such as novel agents - identified using such assay screening methods. The present invention also relates to the use of such agents as antiviral agents.

In particular, the present invention relates *inter alia* to an assay method for identifying an agent that can affect the activity or expression of a nucleotide sequence or the expression product (EP) thereof, the assay method comprising contacting an agent with: a nucleotide sequence coding for a serine/threonine phosphatase; and/or the EP thereof (i.e. a serine/threonine phosphatase); and determining whether the agent affects the activity or expression of the nucleotide sequence and/or the EP.

In a preferred embodiment, the present invention relates *inter alia* to an assay method for identifying an agent that can affect the activity or expression of a nucleotide sequence or the expression product (EP) thereof, the assay method comprising contacting an agent with: a nucleotide sequence coding for a serine/threonine phosphatase; and/or the EP thereof (i.e. a serine/threonine phosphatase); determining whether the agent affects the activity or expression of the nucleotide sequence and/or the EP; and wherein the EP is PP1 or an active sub-unit thereof.

In a preferred embodiment, the present invention relates *inter alia* to an assay method for identifying an agent that can affect the activity or expression of a nucleotide sequence or the expression product (EP) thereof, the assay method comprising contacting an agent with: a nucleotide sequence coding for a serine/threonine phosphatase; and/or the EP thereof (i.e. a serine/threonine phosphatase); determining whether the agent affects the activity or expression of the nucleotide sequence and/or the EP; and wherein the EP is PP1α and/or the targeting sub-unit normally associated with PP1α.

In a more preferred embodiment, the present invention relates *inter alia* to an assay method for identifying an agent that can affect the activity or expression of a nucleotide sequence or the expression product (EP) thereof, the assay method comprising contacting an agent with: a nucleotide sequence coding for a serine/threonine phosphatase; and/or the EP thereof (i.e. a serine/threonine phosphatase); determining whether the agent affects the activity or expression of the nucleotide sequence and/or the EP; wherein the EP is PP1α and/or the targeting sub-unit normally associated with PP1α; and wherein the assay is to screen for anti-viral agents.

In a more preferred embodiment, the present invention relates *inter alia* to an assay method for identifying an agent that can affect the activity or expression of a nucleotide sequence or the expression product (EP) thereof, the assay method comprising contacting an agent with: a nucleotide sequence coding for a serine/threonine phosphatase; and/or the EP thereof (i.e. a serine/threonine phosphatase); determining whether the agent affects the activity or expression of the nucleotide sequence and/or the EP; wherein the EP is PP1α and/or the targeting sub-unit normally associated with PP1α; and wherein the assay is to screen for anti-viral agents but not for anti-HSV agents.

In a more preferred embodiment, the present invention relates *inter alia* to an assay method for identifying an agent that can affect the activity or expression of a nucleotide sequence or the expression product (EP) thereof, the assay method comprising contacting an agent with: a nucleotide sequence coding for a serine/threonine phosphatase; and/or the EP thereof (i.e. a serine/threonine phosphatase); determining whether the agent affects the activity or expression of the nucleotide sequence and/or the EP; wherein the EP is PP1α and/or the targeting sub-unit normally associated with PP1α; and wherein the assay is to screen for agents that can affect the activity or activation of at least P68.

In a more preferred embodiment, the present invention relates *inter alia* to an assay method for identifying an agent that can affect the activity or expression of a nucleotide sequence or the expression product (EP) thereof, the assay method comprising contacting an agent with: a nucleotide sequence coding for a serine/threonine phosphatase; and/or the EP thereof (i.e. a serine/threonine phosphatase); determining whether the agent affects the activity or expression of the nucleotide sequence and/or the EP; wherein the EP is PP1α and/or the targeting sub-unit normally associated with PP1α; and wherein the assay is to screen for agents that can affect the activity or activation of P68 alone.

In a more preferred embodiment, the present invention relates *inter alia* to an assay method for identifying an agent that can affect the activity or expression of a nucleotide sequence or the expression product (EP) thereof, the assay method comprising contacting an agent with: a nucleotide sequence coding for a serine/threonine phosphatase; and/or the EP thereof (i.e. a serine/threonine phosphatase); determining whether the agent affects the activity or expression of the nucleotide sequence and/or the EP; wherein the EP is PP1α and/or the targeting sub-unit normally associated with PP1α; and wherein the agent is not okadaic acid.

Further preferred embodiments of the present invention also include combinations of these preferred and more preferred embodiments.

The present invention will now be described by way of example. Reference has already been made to the following Figures:
Figure 1 which presents a schematic diagram; and
Figure 2 which presents a schematic diagram.

### EXPERIMENTAL SECTION

### Example 1

### In vitro Screening method: p68/PP1 ELISA

p68 (1 µg/ml, 100 µl/well), partially purified as described previously (Barber *et al.* 1991, *Biochemistry* 30:10356), is immobilised in each well of a microtiter plate (Nunc-Immuno MaxiSorb). After incubation for 2.5 hours at 37°C, the plate is washed three times with a solution of 0.1% Tween-20 in phosphate buffered saline (Tween/PBS). To reduce non specific binding, cells are then blocked for 2 hours at 37°C with 200 µl/well of a solution containing 0.1% bovine serum albumin (BSA), 0.1% Tween-20 in PBS. Excess BSA is then removed by washing three times with Tween/PBS. Binding reactions are performed by adding a solution of protein phosphatase 1 (1 µg/ml), partially purified as described previously (Moorhead *et al.,* 1994, *FEBS Lett* 12:46), containing various concentrations of the test inhibitor (typically 5-10 µM). After 1 hour at 37°C, unbound PP1 is removed by 3-4 washes with PBS. Bound PP1 can then be quantitated by liquid scintillation if PP1 labelled with radioactive isotopes I¹²⁵ or H³ is used. Alternatively, rabbit polyclonal anti-PP1 serum, appropriately diluted (typically 1/500 - 1/1000), is added and allowed to bind to PP1 for an hour at room temperature. Anti-rabbit IgG antibody, conjugated to alkaline phosphatase, is then added, and after a final wash step, bound PP1 is quantitated using commercially available chromogenic or fluorogenic substrates for alkaline phosphatase. Typical control reactions during the screen include the following: a) a control reaction with p68 but no PP1; b) a control reaction with a standard PP1 inhibitor but no test compound; and c) a control reaction with neither test compound nor inhibitor.

### Example 2

### In vitro Screening method: p68/PP1α ELISA

Example 1 is repeated but wherein PP1α is used instead of PP1.

### Example 3

### In vitro Screening method: p68/targetting sub-unit normally associated with PP1α ELISA

Example 1 is repeated but wherein the targetting sub-unit normally associated with PP1α is used instead of PP1.

### Example 4

### In vitro Screening method: p68/combination of PP1α and targetting sub-unit normally associated with PP1α ELISA

Example 1 is repeated but wherein the combination of PP1α when bound to the targetting sub-unit normally associated with PP1α is used instead of PP1.

### Example 5

### Anti-Viral Studies

Compounds identified by any one or more of Examples 1 to 4 are then used as anti-viral agents according to any one or more of the above mentioned references.

Thus, in summation, the present invention relates to assay methods for screening for agents useful for treatment of viral infection. The present invention also relates to agents - such as novel agents - identified using such assay screening methods. The present invention also relates to the use of such agents as antiviral agents.

Hence, the present invention provides an assay method for identifying an agent that can affect the activity or expression of a nucleotide sequence or the expression product (EP) thereof, the assay method comprising contacting an agent with: a nucleotide sequence coding for a serine/threonine phosphatase; and/or the EP thereof; and determining whether the agent affects the activity or expression of the nucleotide sequence and/or the EP.

The present invention also provides an assay method for identifying an agent that can affect serine/threonine phosphatase activity or expression thereof, the assay method comprising contacting an agent with an expression product (EP) which is a serine/threonine phosphatase or a nucleotide sequence encoding same; and determining whether the agent binds to or otherwise interacts with and activates or inhibits an activity of the EP; wherein the presence or absence of a signal generated from the binding or interaction of the agent with the EP enzyme or nucleotide sequence encoding same is indicative that the agent can affect the EP activity or expression.

Preferably, the present invention provides an assay method for identifying an anti-viral agent that can affect the activity or expression of a nucleotide sequence or the expression product (EP) thereof, the assay method comprising contacting an agent with: a nucleotide sequence coding for a serine/threonine phosphatase; and/or the EP thereof; and determining whether the agent affects the activity or expression of the nucleotide sequence and/or the EP.

Preferably, the present invention also provides an assay method for identifying an antiviral agent that can affect serine/threonine phosphatase activity or expression thereof, the assay method comprising contacting an agent with an expression product (EP) which is a serine/threonine phosphatase or a nucleotide sequence encoding same; and determining whether the agent binds to or otherwise interacts with and activates or inhibits an activity of the EP; wherein the presence or absence of a signal generated from the binding or interaction of the agent with the EP enzyme or nucleotide sequence encoding same is indicative that the agent can affect the EP activity or expression.

More preferably, the present invention provides an assay method for identifying an anti-viral agent that can affect the activity or expression of a nucleotide sequence or the expression product (EP) thereof, the assay method comprising contacting an agent with: a nucleotide sequence coding for a serine/threonine phosphatase; and/or the EP thereof; and determining whether the agent affects the activity or expression of the nucleotide sequence and/or the EP; wherein the EP is PP1α and/or the targeting sub-unit normally associated with PP1α.

More preferably, the present invention also provides an assay method for identifying an antiviral agent that can affect serine/threonine phosphatase activity or expression thereof, the assay method comprising contacting an agent with an expression product (EP) which is a serine/threonine phosphatase or a nucleotide sequence encoding same; and determining whether the agent binds to or otherwise interacts with and activates or inhibits an activity of the EP; wherein the presence or absence of a signal generated from the binding or interaction of the agent with the EP enzyme or nucleotide sequence encoding same is indicative that the agent can affect the EP activity or expression; wherein the EP is PP1α and/or the targeting sub-unit normally associated with PP1α.

The present invention also provides a modulator of an interaction between a serine/threonine phosphatase and PKR-P and/or eIF2α-P, wherein the modulator is capable of exhibiting anti-viral effects.

In a preferred aspect, the present invention provides a modulator of an interaction between PP1α and/or the targeting sub-unit normally associated with PP1α and PKR-P and/or eIF2α-P, wherein the modulator is capable of exhibiting anti-viral effects.

All publications mentioned in the above specification are herein incorporated by reference. Various modifications and variations of the described methods and system of the invention will be apparent to those skilled in the art without departing from the scope and spirit of the invention. Although the invention has been described in connection with specific preferred embodiments, it should be understood that the invention as claimed should not be unduly limited to such specific embodiments. Indeed, various modifications of the described modes for carrying out the invention which are obvious to those skilled in molecular biology or related fields are intended to be within the scope of the following claims.

### References

Chebath, J., P. Benech, *et al.* (1987). "Constitutive expression of (2'-5') oligo A synthetase confers resistance to picornavirus infection." Nature **330** (6148): 587-8.

Clemens, M. J. and A. Elia (1997). "The double-stranded RNA-dependent protein kinase PKR: structure and function." J Interferon Cytokine Res **17**(9): 503-24.

Gupta, V., A. K. Ogawa, *et al.* (1997). "A model for binding of structurally diverse natural product inhibitors of protein phosphatases PP1 and PP2A." J Med Chem **40**(20): 3199-206.

He, B., M. Gross, *et al.* (1997). "The gamma(1)34.5 protein of herpes simplex virus 1 complexes with protein phosphatase lalpha to dephosphorylate the alpha subunit of the eukaryotic translation initiation factor 2 and preclude the shutoff of protein synthesis by double-stranded RNA-activated protein kinase." Proc Natl Acad Sci U S A **94**(3): 843-8.

Vilcek, J. and G. C. Sen (1996). Interferons and Other Cytokines. Fields Virology. B. N. Fields, D. M. Knipe and P. M. Howley. Philadelphia, Raven Publishers. **1**: 375-399.

Yang, Y. L., L. F. Reis, *et al.* (1995). "Deficient signaling in mice devoid of double-stranded RNA-dependent protein kinase." Embo J **14**(24): 6095-106.

### Annex to the description

SEQUENCES

SEQ ID NO. 1

1400 bp mRNA
DEFINITION protein phosphatase type 1 catalytic subunit [human, mRNA, 1400 nt
SOURCE human.
ORGANISM Homo sapiens
Eukaryotae; mitochondrial eukaryotes; Metazoa; Chordata;
Vertebrata; Eutheria; Primates; Catarrhini; Hominidae; Homo.
FEATURES Location/Qualifiers
source 1..1400
/organism="Homo sapiens"
/db_xref="taxon:9606"
gene 12..1037
/gene="protein phosphatase type 1 catalytic subunit, PP-1
alpha 2"
CDS 12..1037
/gene="protein phosphatase type 1 catalytic subunit, PP-1
alpha 2"
/note="This sequence comes from Fig. 5; PP-1 alpha 2"
/codon_start=1
/product="protein phosphatase type 1 catalytic subunit"
/db_xref="PID:g298964"

1 gcggcgccgc catgtccgac agcgagaagc tcaacctgga ctcgatcatc gggcgcctgc
61 tggaagggtc cagggtcctg acaccccatt gcgccccagt gcagggctcg cggcctggca
121 agaatgtaca gctgacagag aacgagatcc gcggtctgtg cctgaaatcc cgggagattt
181 ttctgagcca gcccattctt ctggagctgg aggcacccct caagatctgc ggtgacatac
241 acggccagta ctacgacctt ctgcgactat ttgagtatgg cggtttccct cccgagagca
301 actacctctt tctgggggac tatgtggaca ggggcaagca gtccttggag accatctgcc
361 tgctgctggc ctataagatc aagtaccccg agaacttctt cctgctccgt gggaaccacg
421 agtgtgccag catcaaccgc atctatggtt tctacgatga gtgcaagaga cgctacaaca
481 tcaaactgtg gaaaaccttc actgactgct tcaactgcct gcccatcgcg gccatagtgg
541 acgaaaagat cttctgctgc cacggaggcc tgtccccgga cctgcagtct atggagcaga
601 ttcggcggat catgcggccc acagatgtgc ctgaccaggg cctgctgtgt gacctgctgt
661 ggtctgaccc tgacaaggac gtgcagggct ggggcgagaa cgaccgtggc gtctctttta
721 cctttggagc cgaggtggtg gccaagttcc tccacaagca cgacttggac ctcatctgcc
781 gagcacacca ggtggtagaa gacggctatg agttctttgc caagcggcag ctggtgacac
841 ttttctcagc tcccaactac tgtggcgagt ttgacaatgc tggcgccatg atgagtgtgg
901 acgagaccct catgtgctct ttccagatcc tcaagcccgc cgacaagaac aaggggaagt
961 acgggcagtt cagtggcctg aaccctggag gccgacccat caccccaccc cgcaattccg
1021 ccaaagccaa gaaatagccc ccgcacacca ccctgtgccc cagatgatgg attgattgta
1081 cagaaatcat gctgccatgc tggggggggg tcaccccgac ccctaaggcc cacctgtcac
1141 ggggaacatg gagccttggt gtatttttct ttcttttttt taatgaatca atagcagcgt
1201 ccagtccccc agggctgctt cctgcctgca cctgcggtac tgtgagcagg atcctggggc
1261 cgaggctgca gctcagggca acggcaggcc aggtcgtggg tctccagccg tgcttggcct
1321 caggctggca gcccggatcc tggggcaacc catctggtct cttgaataaa ggtcaaagct
1381 ggattctcaa aaaaaaaaaa

BASE COUNT:

302 a
415 c
392 g
291 t

SEQ ID NO.2

SOURCE human.
ORGANISM Homo sapiens
Eukaryotae; mitochondrial eukaryotes; Metazoa; Chordata;
Vertebrata; Eutheria; Primates; Catarrhini; Hominidae; Homo.

MSDSEKLNLDSIIGRLLEGSRVLTPHCAPVQGSRPGKNVQLTEN
EIRGLCLKSREIFLSQPILLELEAPLKICGDIHGQYYDLLRLFEYGGFPPESNYLFLG
DYVDRGKQSLETICLLLAYKIKYPENFFLLRGNHECASINRIYGFYDECKRRYNIKLW
KTFTDCFNCLPIAAIVDEKIFCCHGGLSPDLQSMEQIRRIMRPTDVPDQGLLCDLLWS
DPDKDVQGWGENDRGVSFTFGAEVVAKFLHKHDLDLICRAHQVVEDGYEFFAKRQLVT
LFSAPNYCGEFDNAGAMMSVDETLMCSFQILKPADKNKGKYGQFSGLNPGGRPITPPR
NSAKAKK

SEQ ID NO.3

LOCUS HSU83981 2331 bp mRNA PRI 07-JUL-1998
DEFINITION Homo sapiens apoptosis associated protein (GADD34) mRNA, complete
cds.
ACCESSION U83981
NID g3258617
VERSION U83981.1 GI:3258617
KEYWORDS .
SOURCE human.
ORGANISM Homo sapiens
Eukaryota; Metazoa; Chordata; Craniata; Vertebrata; Mammalia;
Eutheria; Primates; Catarrhini; Hominidae; Homo.
REFERENCE 1 (bases 1 to 2331)
AUTHORS Hollander,M.C., Zhan,Q., Bae,I. and Fornace,A.J. Jr.
TITLE Mammalian GADD34, an apoptosis- and DNA damage-inducible gene
JOURNAL J. Biol. Chem. 272 (21), 13731-13737 (1997)
MEDLINE 97298078
REFERENCE 2 (bases 1 to 2331)
AUTHORS Hollander,M.C. and Fornace,A.J. Jr.
TITLE Direct Submission
JOURNAL Submitted (07-JAN-1997) LMP, NIH, Bldg.37, Room 5C09, Bethesda, MD
20892, USA
FEATURES Location/Qualifiers
source 1..2331
/organism="Homo sapiens"
/db_xref="taxon:9606"
/chromosome="19"
/map="19q13.2"
gene 1..2331

/gene="GADD34"
/note="growth arrest and DNA damage-inducible gene 34"

/gene="GADD34"
/note="similar to HSV-1 ICP34.5 protein, SwissProt
Accession Number P36313 and ASFV DP71L gene deposited
under GenBank Accession Number U18466"
ORIGIN
1 cccagttgtt gatcttatgc aagacgctgc acgaccccgc gcccgcttgt cgccacggca
61 cttgaggcag ccggagatac tctgagttac tcggagcccg acgcctgagg gtgagatgaa
121 cgcgctggcc tccctaaccg tccggacctg tgatcgcttc tggcagaccg aaccggcgct
181 cctgcccccg gggtgacgcg cagctcccag ccgcccagac acatggcccc aggccaagca
241 ccccatcagg ctaccccgtg gagggatgcc caccctttct tcctcctgtc cccagtgatg
301 ggcctcctca gccgcgcctg gagccgcctg aggggcctgg gacctctaga gccctggctg
361 gtggaagcag taaaaggagc agctctggta gaagctggcc tggagggaga agctaggact
421 cctctggcaa tcccccatac cccttggggc agacgccctg aagaggaggc tgaagacagt
481 ggaggccctg gagaggacag agaaacactg gggctgaaaa ccagcagttc ccttcctgaa
541 gcctggggac ttttggatga tgatgatggc atgtatggtg agcgagaggc aaccagtgtc
601 cctagagggc agggaagtca atttgcagat ggccagcgtg ctcccctgtc tcccagcctt
661 ctgataagga cactgcaagg ttctgataag aacccagggg aggagaaagc cgaggaagag
721 ggagttgctg aagaggaggg agttaacaag ttctcttatc caccatcaca ccgggagtgt
781 tgtccagccg tggaggagga ggacgatgaa gaagctgtaa agaaagaagc tcacagaacc
841 tctacttctg ccttgtctcc aggatccaag cccagcactt gggtgtcttg cccaggggag
901 gaagagaatc aagccacgga ggataaaaga acagaaagaa gtaaaggagc caggaagacc
961 tccgtgtccc cccgatcttc aggctccgac cccaggtcct gggagtatcg ttcaggagag
1021 gcgtccgagg agaaggagga aaaggcacac aaagaaactg ggaaaggaga agctgcccca
1081 gggccgcaat cctcagcccc agcccagagg ccccagctca agtcctggtg gtgccaaccc
1141 agtgatgaag aggagggtga ggtcaaggct ttgggggcag ctgagaagga tggagaagct
1201 gagtgtcctc cctgcatccc cccaccaagt gccttcctga aggcctgggt gtattggcca
1261 ggagaggaca cagaggaaga ggaagatgag gaagaagatg aggacagtga ctctggatca
1321 gatgaggaag agggagaagc tgaggcttcc tcttccactc ctgctacagg tgtcttcttg
1381 aagtcctggg tctatcagcc aggagaggac acagaggagg aggaagatga ggacagtgat
1441 acaggatcag ccgaggatga aagagaagct gagacttctg cttccacacc ccctgcaagt
1501 gctttcttga aggcctgggt gtatcggcca ggagaggaca cggaggagga ggaagatgag
1561 gatgtggata gtgaggataa ggaagatgat tcagaagcag ccttgggaga agctgagtca
1621 gacccacatc cctcccaccc ggaccagagg gcccacttca ggggctgggg atatcgacct
1681 ggaaaagaga cagaggaaga ggaagctgct gaggactggg gagaagctga gccctgcccc
1741 ttccgagtgg ccatctatgt acctggagag aagccaccgc ctccctgggc tcctcctagg
1801 ctgcccctcc gactgcaaag gcggctcaag cgcccagaaa cccctactca tgatccggac
1861 cctgagactc ccctaaaggc cagaaaggtg cgcttctccg agaaggtcac tgtccatttc
1921 ctggctgtct gggcagggcc ggcccaggcc gcccgccagg gcccctggga gcagcttgct
1981 cgggatcgca gccgcttcgc acgccgcatc acccaggccc aggaggagct gagcccctgc
2041 ctcacccctg ctgcccgggc cagagcctgg gcacgcctca ggaacccacc tttagccccc
2101 atccctgccc tcacccagac cttgccttcc tcctctgtcc cttcgtcccc agtccagacc
2161 acgcccttga gccaagctgt ggccacacct tcccgctcgt ctgctgctgc agcggctgcc
2221 ctggacctca gtgggaggcg tggctgagac caactggttt gcctataatt tattaactat
2281 ttattttttc taagtgtggg tttatataag gaataaagcc ttttgatttg t

BASE COUNT

543 a
666 c
712 g
410 t

SEQUENCE LISTING

<110> Pfizer Limited

<120> Assay

<130> Application number: 99307236.2

<140>
<141>

<160> 4

<170> PatentIn Ver. 2.0

<210> 1
<211> 1400
<212> DNA
<213> Homo sapiens

<400> 1
gcggcgccgc catgtccgac agcgagaagc tcaacctgga ctcgatcatc gggcgcctgc 60
tggaagggtc cagggtcctg acaccccatt gcgccccagt gcagggctcg cggcctggca 120
agaatgtaca gctgacagag aacgagatcc gcggtctgtg cctgaaatcc cgggagattt 180
ttctgagcca gcccattctt ctggagctgg aggcacccct caagatctgc ggtgacatac 240
acggccagta ctacgacctt ctgcgactat ttgagtatgg cggtttccct cccgagagca 300
actacctctt tctgggggac tatgtggaca ggggcaagca gtccttggag accatctgcc 360
tgctgctggc ctataagatc aagtaccccg agaacttctt cctgctccgt gggaaccacg 420
agtgtgccag catcaaccgc atctatggtt tctacgatga gtgcaagaga cgctacaaca 480
tcaaactgtg gaaaaccttc actgactgct tcaactgcct gcccatcgcg gccatagtgg 540
acgaaaagat cttctgctgc cacggaggcc tgtccccgga cctgcagtct atggagcaga 600
ttcggcggat catgcggccc acagatgtgc ctgaccaggg cctgctgtgt gacctgctgt 660
ggtctgaccc tgacaaggac gtgcagggct ggggcgagaa cgaccgtggc gtctctttta 720
cctttggagc cgaggtggtg gccaagttcc tccacaagca cgacttggac ctcatctgcc 780
gagcacacca ggtggtagaa gacggctatg agttctttgc caagcggcag ctggtgacac 840
ttttctcagc tcccaactac tgtggcgagt ttgacaatgc tggcgccatg atgagtgtgg 900
acgagaccct catgtgctct ttccagatcc tcaagcccgc cgacaagaac aaggggaagt 960
acgggcagtt cagtggcctg aaccctggag gccgacccat caccccaccc cgcaattccg 1020
ccaaagccaa gaaatagccc ccgcacacca ccctgtgccc cagatgatgg attgattgta 1080
cagaaatcat gctgccatgc tggggggggg tcaccccgac ccctaaggcc cacctgtcac 1140
ggggaacatg gagccttggt gtatttttct ttcttttttt taatgaatca atagcagcgt 1200
ccagtccccc agggctgctt cctgcctgca cctgcggtac tgtgagcagg atcctggggc 1260
cgaggctgca gctcagggca acggcaggcc aggtcgtggg tctccagccg tgcttggcct 1320
caggctggca gcccggatcc tggggcaacc catctggtct cttgaataaa ggtcaaagct 1380
ggattctcaa aaaaaaaaaa 1400

<210> 2
<211> 341

<212> PRT
<213> Homo sapiens

<400> 2
Met Ser Asp Ser Glu Lys Leu Asn Leu Asp Ser Ile Ile Gly Arg Leu
1               5                   10                  15

Leu Glu Gly Ser Arg Val Leu Thr Pro His Cys Ala Pro Val Gln Gly
            20                  25                  30

Ser Arg Pro Gly Lys Asn Val Gln Leu Thr Glu Asn Glu Ile Arg Gly
        35                  40                  45

Leu Cys Leu Lys Ser Arg Glu Ile Phe Leu Ser Gln Pro Ile Leu Leu
    50                  55                  60

Glu Leu Glu Ala Pro Leu Lys Ile Cys Gly Asp Ile His Gly Gln Tyr
65                  70                  75                  80

Tyr Asp Leu Leu Arg Leu Phe Glu Tyr Gly Gly Phe Pro Pro Glu Ser
                85                  90                  95

Asn Tyr Leu Phe Leu Gly Asp Tyr Val Asp Arg Gly Lys Gln Ser Leu
            100                 105                 110

Glu Thr Ile Cys Leu Leu Leu Ala Tyr Lys Ile Lys Tyr Pro Glu Asn
        115                 120                 125

Phe Phe Leu Leu Arg Gly Asn His Glu Cys Ala Ser Ile Asn Arg Ile
    130                 135                 140

Tyr Gly Phe Tyr Asp Glu Cys Lys Arg Arg Tyr Asn Ile Lys Leu Trp
145                 150                 155                 160

Lys Thr Phe Thr Asp Cys Phe Asn Cys Leu Pro Ile Ala Ala Ile Val
                165                 170                 175

Asp Glu Lys Ile Phe Cys Cys His Gly Gly Leu Ser Pro Asp Leu Gln
            180                 185                 190

Ser Met Glu Gln Ile Arg Arg Ile Met Arg Pro Thr Asp Val Pro Asp
        195                 200                 205

Gln Gly Leu Leu Cys Asp Leu Leu Trp Ser Asp Pro Asp Lys Asp Val
    210                 215                 220

Gln Gly Trp Gly Glu Asn Asp Arg Gly Val Ser Phe Thr Phe Gly Ala
225                 230                 235                 240

Glu Val Val Ala Lys Phe Leu His Lys His Asp Leu Asp Leu Ile Cys
                245                 250                 255

Arg Ala His Gln Val Val Glu Asp Gly Tyr Glu Phe Phe Ala Lys Arg
            260                 265                 270

Gln Leu Val Thr Leu Phe Ser Ala Pro Asn Tyr Cys Gly Glu Phe Asp
        275                 280                 285

Asn Ala Gly Ala Met Met Ser Val Asp Glu Thr Leu Met Cys Ser Phe
    290                 295                 300

Gln Ile Leu Lys Pro Ala Asp Lys Asn Lys Gly Lys Tyr Gly Gln Phe
305                 310                 315                 320

Ser Gly Leu Asn Pro Gly Gly Arg Pro Ile Thr Pro Pro Arg Asn Ser
                325                 330                 335

Ala Lys Ala Lys Lys
            340

<210> 3
<211> 2331
<212> DNA
<213> Homo sapiens

<400> 3
cccagttgtt gatcttatgc aagacgctgc acgaccccgc gcccgcttgt cgccacggca 60
cttgaggcag ccggagatac tctgagttac tcggagcccg acgcctgagg gtgagatgaa 120
cgcgctggcc tccctaaccg tccggacctg tgatcgcttc tggcagaccg aaccggcgct 180
cctgcccccg gggtgacgcg cagctcccag ccgcccagac acatggcccc aggccaagca 240
ccccatcagg ctaccccgtg gagggatgcc caccctttct tcctcctgtc cccagtgatg 300
ggcctcctca gccgcgcctg gagccgcctg aggggcctgg gacctctaga gccctggctg 360
gtggaagcag taaaaggagc agctctggta gaagctggcc tggagggaga agctaggact 420
cctctggcaa tcccccatac cccttggggc agacgccctg aagaggaggc tgaagacagt 480
ggaggccctg gagaggacag agaaacactg gggctgaaaa ccagcagttc ccttcctgaa 540
gcctggggac ttttggatga tgatgatggc atgtatggtg agcgagaggc aaccagtgtc 600
cctagagggc agggaagtca atttgcagat ggccagcgtg ctcccctgtc tcccagcctt 660
ctgataagga cactgcaagg ttctgataag aacccagggg aggagaaagc cgaggaagag 720
ggagttgctg aagaggaggg agttaacaag ttctcttatc caccatcaca ccgggagtgt 780
tgtccagccg tggaggagga ggacgatgaa gaagctgtaa agaaagaagc tcacagaacc 840
tctacttctg ccttgtctcc aggatccaag cccagcactt gggtgtcttg cccaggggag 900
gaagagaatc aagccacgga ggataaaaga acagaaagaa gtaaaggagc caggaagacc 960
tccgtgtccc cccgatcttc aggctccgac cccaggtcct gggagtatcg ttcaggagag 1020
gcgtccgagg agaaggagga aaaggcacac aaagaaactg ggaaaggaga agctgcccca 1080
gggccgcaat cctcagcccc agcccagagg ccccagctca agtcctggtg gtgccaaccc 1140

agtgatgaag aggagggtga ggtcaaggct ttgggggcag ctgagaagga tggagaagct 1200
gagtgtcctc cctgcatccc cccaccaagt gccttcctga aggcctgggt gtattggcca 1260
ggagaggaca cagaggaaga ggaagatgag gaagaagatg aggacagtga ctctggatca 1320
gatgaggaag agggagaagc tgaggcttcc tcttccactc ctgctacagg tgtcttcttg 1380
aagtcctggg tctatcagcc aggagaggac acagaggagg aggaagatga ggacagtgat 1440
acaggatcag ccgaggatga aagagaagct gagacttctg cttccacacc ccctgcaagt 1500
gctttcttga aggcctgggt gtatcggcca ggagaggaca cggaggagga ggaagatgag 1560
gatgtggata gtgaggataa ggaagatgat tcagaagcag ccttgggaga agctgagtca 1620
gacccacatc cctcccaccc ggaccagagg gcccacttca ggggctgggg atatcgacct 1680
ggaaaagaga cagaggaaga ggaagctgct gaggactggg gagaagctga gccctgcccc 1740
ttccgagtgg ccatctatgt acctggagag aagccaccgc ctccctgggc tcctcctagg 1800
ctgcccctcc gactgcaaag gcggctcaag cgcccagaaa cccctactca tgatccggac 1860
cctgagactc ccctaaaggc cagaaaggtg cgcttctccg agaaggtcac tgtccatttc 1920
ctggctgtct gggcagggcc ggcccaggcc gcccgccagg gcccctggga gcagcttgct 1980
cgggatcgca gccgcttcgc acgccgcatc acccaggccc aggaggagct gagcccctgc 2040
ctcacccctg ctgcccgggc cagagcctgg gcacgcctca ggaacccacc tttagccccc 2100
atccctgccc tcacccagac cttgccttcc tcctctgtcc cttcgtcccc agtccagacc 2160
acgcccttga gccaagctgt ggccacacct tcccgctcgt ctgctgctgc agcggctgcc 2220
ctggacctca gtgggaggcg tggctgagac caactggttt gcctataatt tattaactat 2280
ttattttttc taagtgtggg tttatataag gaataaagcc ttttgatttg t 2331

<210> 4
<211> 674
<212> PRT
<213> Homo sapiens

<400> 4
Met Ala Pro Gly Gln Ala Pro His Gln Ala Thr Pro Trp Arg Asp Ala
1               5                   10                  15

His Pro Phe Phe Leu Leu Ser Pro Val Met Gly Leu Leu Ser Arg Ala
            20                  25                  30

Trp Ser Arg Leu Arg Gly Leu Gly Pro Leu Glu Pro Trp Leu Val Glu
        35                  40                  45

Ala Val Lys Gly Ala Ala Leu Val Glu Ala Gly Leu Glu Gly Glu Ala
    50                  55                  60

Arg Thr Pro Leu Ala Ile Pro His Thr Pro Trp Gly Arg Arg Pro Glu
65                  70                  75                  80

Glu Glu Ala Glu Asp Ser Gly Gly Pro Gly Glu Asp Arg Glu Thr Leu
                85                  90                  95

Gly Leu Lys Thr Ser Ser Ser Leu Pro Glu Ala Trp Gly Leu Leu Asp
            100                 105                 110

Asp Asp Asp Gly Met Tyr Gly Glu Arg Glu Ala Thr Ser Val Pro Arg
        115                 120                 125

Gly Gln Gly Ser Gln Phe Ala Asp Gly Gln Arg Ala Pro Leu Ser Pro
    130                 135                 140

Ser Leu Leu Ile Arg Thr Leu Gln Gly Ser Asp Lys Asn Pro Gly Glu
145                 150                 155                 160

Glu Lys Ala Glu Glu Glu Gly Val Ala Glu Glu Glu Gly Val Asn Lys
                165                 170                 175

Phe Ser Tyr Pro Pro Ser His Arg Glu Cys Cys Pro Ala Val Glu Glu
            180                 185                 190

Glu Asp Asp Glu Glu Ala Val Lys Lys Glu Ala His Arg Thr Ser Thr
        195                 200                 205

Ser Ala Leu Ser Pro Gly Ser Lys Pro Ser Thr Trp Val Ser Cys Pro
    210                 215                 220

Gly Glu Glu Glu Asn Gln Ala Thr Glu Asp Lys Arg Thr Glu Arg Ser
225                 230                 235                 240

Lys Gly Ala Arg Lys Thr Ser Val Ser Pro Arg Ser Ser Gly Ser Asp
                245                 250                 255

Pro Arg Ser Trp Glu Tyr Arg Ser Gly Glu Ala Ser Glu Glu Lys Glu
            260                 265                 270

Glu Lys Ala His Lys Glu Thr Gly Lys Gly Glu Ala Ala Pro Gly Pro
        275                 280                 285

Gln Ser Ser Ala Pro Ala Gln Arg Pro Gln Leu Lys Ser Trp Trp Cys
    290                 295                 300

Gln Pro Ser Asp Glu Glu Glu Gly Glu Val Lys Ala Leu Gly Ala Ala
305                 310                 315                 320

Glu Lys Asp Gly Glu Ala Glu Cys Pro Pro Cys Ile Pro Pro Pro Ser
                325                 330                 335

Ala Phe Leu Lys Ala Trp Val Tyr Trp Pro Gly Glu Asp Thr Glu Glu
            340                 345                 350

Glu Glu Asp Glu Glu Glu Asp Glu Asp Ser Asp Ser Gly Ser Asp Glu
        355                 360                 365

Glu Glu Gly Glu Ala Glu Ala Ser Ser Ser Thr Pro Ala Thr Gly Val
    370                 375                 380

Phe Leu Lys Ser Trp Val Tyr Gln Pro Gly Glu Asp Thr Glu Glu Glu
385                 390                 395                 400

Glu Asp Glu Asp Ser Asp Thr Gly Ser Ala Glu Asp Glu Arg Glu Ala
                405                 410                 415

Glu Thr Ser Ala Ser Thr Pro Pro Ala Ser Ala Phe Leu Lys Ala Trp
            420                 425                 430

Val Tyr Arg Pro Gly Glu Asp Thr Glu Glu Glu Glu Asp Glu Asp Val
        435                 440                 445

Asp Ser Glu Asp Lys Glu Asp Asp Ser Glu Ala Ala Leu Gly Glu Ala
    450                 455                 460

Glu Ser Asp Pro His Pro Ser His Pro Asp Gln Arg Ala His Phe Arg
465                 470                 475                 480

Gly Trp Gly Tyr Arg Pro Gly Lys Glu Thr Glu Glu Glu Glu Ala Ala
                485                 490                 495

Glu Asp Trp Gly Glu Ala Glu Pro Cys Pro Phe Arg Val Ala Ile Tyr
            500                 505                 510

Val Pro Gly Glu Lys Pro Pro Pro Pro Trp Ala Pro Pro Arg Leu Pro
        515                 520                 525

Leu Arg Leu Gln Arg Arg Leu Lys Arg Pro Glu Thr Pro Thr His Asp
    530                 535                 540

Pro Asp Pro Glu Thr Pro Leu Lys Ala Arg Lys Val Arg Phe Ser Glu
545                 550                 555                 560

Lys Val Thr Val His Phe Leu Ala Val Trp Ala Gly Pro Ala Gln Ala
                565                 570                 575

Ala Arg Gln Gly Pro Trp Glu Gln Leu Ala Arg Asp Arg Ser Arg Phe
            580                 585                 590

Ala Arg Arg Ile Thr Gln Ala Gln Glu Glu Leu Ser Pro Cys Leu Thr
        595                 600                 605

Pro Ala Ala Arg Ala Arg Ala Trp Ala Arg Leu Arg Asn Pro Pro Leu
    610                 615                 620

Ala Pro Ile Pro Ala Leu Thr Gln Thr Leu Pro Ser Ser Ser Val Pro
625                 630                 635                 640

Ser Ser Pro Val Gln Thr Thr Pro Leu Ser Gln Ala Val Ala Thr Pro
                645                 650                 655

Ser Arg Ser Ser Ala Ala Ala Ala Ala Ala Leu Asp Leu Ser Gly Arg
            660                 665                 670

Arg Gly

SEQ ID NO.4

LOCUS HSU83981 2331 bp mRNA PRI 07-JUL-1998
DEFINITION Homo sapiens apoptosis associated protein (GADD34) mRNA, complete
cds.
ACCESSION U83981
NID g3258617
VERSION U83981.1 GI:3258617
KEYWORDS .
SOURCE human.
ORGANISM Homo sapiens
Eukaryota; Metazoa; Chordata; Craniata; Vertebrata; Mammalia;
Eutheria; Primates; Catarrhini; Hominidae; Homo.
REFERENCE 1 (bases 1 to 2331)
AUTHORS Hollander,M.C., Zhan,Q., Bae,I. and Fornace,A.J. Jr.
TITLE Mammalian GADD34, an apoptosis- and DNA damage-inducible gene
JOURNAL J. Biol. Chem. 272 (21), 13731-13737 (1997)
MEDLINE 97298078
REFERENCE 2 (bases 1 to 2331)
AUTHORS Hollander,M.C. and Fornace,A.J. Jr.
TITLE Direct Submission
JOURNAL Submitted (07-JAN-1997) LMP, NIH, Bldg.37, Room 5C09, Bethesda, MD
20892, USA
FEATURES Location/Qualifiers
source 1..2331
/organism="Homo sapiens"
/db_xref="taxon:9606"
/chromosome="19"
/map="19q13.2"
gene 1..2331
/gene="GADD34"
/note="growth arrest and DNA damage-inducible gene 34"
CDS 223..2247
/gene="GADD34"
/function="growth arrest"
/note="Gadd34"
/codon_start=1
/product="apoptosis associated protein"
/protein_id="AAC25631.1"
/db_xref="PID:g3258618"
/db_xref="GI:3258618"
/translation=
MAPGQAPHQATPWRDAHPFFLLSPVMGLLSRAWSRLRGLGPLEP
WLVEAVKGAALVEAGLEGEARTPLAIPHTPWGRRPEEEAEDSGGPGEDRETLGLKTSS
SLPEAWGLLDDDDGMYGEREATSVPRGQGSQFADGQRAPLSPSLLIRTLQGSDKNPGE
EKAEEEGVAEEEGVNKFSYPPSHRECCPAVEEEDDEEAVKKEAHRTSTSALSPGSKPS
TWVSCPGEEENQATEDKRTERSKGARKTSVSPRSSGSDPRSWEYRSGEASEEKEEKAH
KETGKGEAAPGPQSSAPAQRPQLKSWWCQPSDEEEGEVKALGAAEKDGEAECPPCIPP
PSAFLKAWVYWPGEDTEEEEDEEEDEDSDSGSDEEEGEAEASSSTPATGVFLKSWVYQ
PGEDTEEEEDEDSDTGSAEDEREAETSASTPPASAFLKAWVYRPGEDTEEEEDEDVDS
EDKEDDSEAALGEAESDPHPSHPDQRAHFRGWGYRPGKETEEEEAAEDWGEAEPCPFR
VAIYVPGEKPPPPWAPPRLPLRLQRRLKRPETPTHDPDPETPLKARKVRFSEKVTVHF
LAVWAGPAQAARQGPWEQLARDRSRFARRITQAQEELSPCLTPAARARAWARLRNPPL
APIPALTQTLPSSSVPSSPVQTTPLSQAVATPSRSSAAAAAALDLSGRRG

## Claims

1. An assay method for identifying an anti-viral agent that can affect the activity or expression of a nucleotide sequence or the expression product (EP) thereof,
the assay method comprising
contacting an agent with:
a nucleotide sequence coding for a serine/threonine phosphatase and/or the EP thereof; and
determining whether the agent affects the activity or expression of the nucleotide sequence and/or the EP.

2. An assay method for identifying an anti-viral agent that can affect serine/threonine phosphatase activity or expression thereof, the assay method comprising
contacting an agent with an expression product (EP) which is a serine/threonine phosphatase or a nucleotide sequence encoding the same; and
determining whether the agent binds to or otherwise interacts with and activates or inhibits an activity of the EP;
wherein the presence or absence of a signal generated from the binding or interaction of the agent with the EP enzyme or nucleotide sequence encoding same is indicative that the agent can affect the EP activity or expression.

3. An assay method according to claim 1 or claim 2 wherein the serine/threonine phosphatase is a mammalian serine/threonine phosphatase.

4. An assay method according to any one of claims 1 to 3 wherein the serine/threonine phosphatase is PP1 or an active sub-unit thereof.

5. An assay method according to any one of claims 1 to 4 wherein the active sub-unit of the serine/threonine phosphatase is at least PP1α and/or the targeting sub-unit normally associated with PP1α, preferably wherein the active sub-unit of the serine/threonine phosphatase is PP1α and the targeting sub-unit normally associated with PP1α.

6. An assay method according to any one of claims 1 to 5 wherein the EP comprises the sequence presented as SEQ ID No. 2 and/or SEQ ID No.4, or a variant, homologue, fragment or derivative thereof.

7. An assay method according to any one of claims 1 to 6 wherein the nucleotide sequence comprises the sequence presented as SEQ ID No. 1 and/or SEQ ID No.3, or a variant, homologue, fragment or derivative thereof.

8. An assay method according to any one of the preceding claims wherein the assay is to screen for agents useful in the treatment of human viral infections.

9. A process comprising the steps of:
(a) performing the assay according to any one of claims 1 to 8;
(b) identifying one or more agents that do affect the EP activity or expression; and
(c) preparing a quantity of those one or more identified agents.

10. An agent identified by the assay according to any one of claims 1 to 8 or the process of claim 9.

11. A method of affecting *in vivo* the serine/threonine phosphatase activity or expression thereof with an agent;
wherein the agent is capable of affecting the serine/threonine phosphatase activity or expression thereof in an *in vitro* assay method;
wherein the *in vitro* assay method is the assay method defined in any one of claims 1 to 8.

12. Use of an agent in the preparation of a pharmaceutical composition for the treatment of a viral disease or condition associated with serine/threonine phosphatase activity or the expression thereof, the agent having an effect on the activity or expression of the serine/threonine phosphatase or the expression thereof when assayed *in vitro* by the assay method according to any one of claims 1 to 8.

13. Use of an agent which has an effect on the activity of a serine/threonine phosphatase or the expression thereof in the preparation of a pharmaceutical composition for the treatment of a viral disease or condition associated with serine/threonine phosphatase activity or the expression thereof.

14. A collection of anti-viral compounds wherein one or more of its members is capable of interacting with a serine/threonine phosphatase or a nucleotide sequence encoding same.

15. A combination comprising the agent of claim 10 and an additional agent therapeutic agent, wherein the additional therapeutic agent is different from the agent of claim 10.

16. A combination according to claim 15 wherein the additional therapeutic agent is capable of inhibiting a viral or host component that affects the PKR pathway.

17. A combination according to claim 16 wherein the viral or host component inhibits or prevents the PKR pathway.

18. A combination according to claim 17 wherein the viral component is NS5A and the host component is P58.

19. A combination according to claim 15 wherein the additional therapeutic agent is an interferon.

20. Use of an anti-viral modulator of PP1 phosphatase activity to inhibit the action of a host component which interferes with the activation of a host-cell interferon-induced, double-stranded RNA-activated protein kinase.

21. Use of an anti-viral modulator of PP1 phosphatase activity to inhibit the action of a host component which interferes with the activation of P68.

22. Use of an anti-viral modulator of PP1 phosphatase activity to inhibit the action of a host component which interferes with the activation of eIF2.

23. Use of an anti-viral modulator of PP1 phosphatase activity to inhibit the action of a host component which interferes with the activation of eIF2α.

24. An assay method as defined in any one of the preceding claims wherein the assay is a cell based assay.

25. A modulator of an interaction between a serine/threonine phosphatase and PKR-P and/or eIF2α-P, wherein the modulator is capable of exhibiting anti-viral effects, wherein the modulator is not okadaic acid.

26. A modulator of an interaction between PP1α and PKR-P and/or eIF2α-P, wherein the modulator is capable of exhibiting anti-viral effects, wherein the modulator is not okadaic acid.

27. An assay method substantially as described herein.
